# EUROPEAN PATENT APPLICATION

(11) **EP 1 365 033 A1**
(43) Date of publication of application: **26.11.2003**
(21) Application number: 02701664.1
(22) Date of filing: 01.03.2002
(51) Int. Cl.: C12Q 1/68, C12Q 1/02, G01N 33/15, C12N 15/09, A61P 35/02

(54) **METHOD OF MOLECULAR DIAGNOSIS OF CHRONIC MYELOGENOUS LEUKEMIA**

(30) Priority: 01.03.2001 JP 2001056438
(71) Applicant: FUJISAWA PHARMACEUTICAL CO., LTD., Osaka-shi Osaka 541-8514 (JP)
(72) Inventor: MANO, Hiroyuki, Utsunomiya-shi, Tochigi 320-0003 (JP)
(74) Representative: Gille Hrabal Struck Neidlein Prop Roos
(86) International application number: JP0201901
(87) International publication number: WO02070747

(57) **Abstract**

A method for detecting chronic myeloid leukemia is provided. The method comprises the step of measuring expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3 gene in a test tissue or a test cell; or determining expression profile of a gene group containing at least one of said genes so as to thereby detect chronic myeloid leukemia.

SEQUENCE LISTING

<110> FUJISAWA PHARMACEUTICAL CO., LTD

<120> METHOD OF MOLECULAR DIAGNOSIS OF CHRONIC MYELOID LEUKEMIA

<130> PH-1505-PCT

<150> JP 2001-56438
<151> 2001-03-01

<160> 23

<170> PatentIn Ver. 2.0

<210> 1
<211> 2581
<212> DNA
<213> Homo sapiens

<220>
<221> CDS
<222> (76)..(1494)

<400> 1
agaagaggaa gatttctgaa gagtgcagct gcctgaaccg agccctgccg aacagctgag 60
aattgcagtg caacc atg agt gag aac aat aag aat tcc ttg gag agc agc 111

Met Ser Glu Asn Asn Lys Asn Ser Leu Glu Ser Ser
1               5                   10
cta cgg caa cta aaa tgc cat ttc acc tgg aac ttg atg gag gga gaa    159
Leu Arg Gln Leu Lys Cys His Phe Thr Trp Asn Leu Met Glu Gly Glu
        15                  20                  25
aac tcc ttg gat gat ttt gaa gac aaa gta ttt tac cgg act gag ttt    207
Asn Ser Leu Asp Asp Phe Glu Asp Lys Val Phe Tyr Arg Thr Glu Phe
    30                  35                  40
cag aat cgt gaa ttc aaa gcc aca atg tgc aac cta ctg gcc tat cta    255
Gln Asn Arg Glu Phe Lys Ala Thr Met Cys Asn Leu Leu Ala Tyr Leu
45                  50                  55                  60
aag cac ctc aaa ggg caa aac gag gca gcc ctg gaa tgc tta cgt aaa    303
Lys His Leu Lys Gly Gln Asn Glu Ala Ala Leu Glu Cys Leu Arg Lys
                65                  70                  75
gct gaa gag tta atc cag caa gag cat gct gac cag gca gaa atc aga    351
Ala Glu Glu Leu Ile Gln Gln Glu His Ala Asp Gln Ala Glu Ile Arg
            80                  85                  90
agt ctg gtc acc tgg gga aac tat gcc tgg gtc tac tat cac atg ggc    399
Ser Leu Val Thr Trp Gly Asn Tyr Ala Trp Val Tyr Tyr His Met Gly
        95                  100                 105
cga ctc tca gac gtt cag att tat gta gac aag gtg aaa cat gtc tgt    447
Arg Leu Ser Asp Val Gln Ile Tyr Val Asp Lys Val Lys His Val Cys
    110                 115                 120
gag aag ttt tcc agt ccc tat aga att gag agt cca gag ctt gac tgt    495
Glu Lys Phe Ser Ser Pro Tyr Arg Ile Glu Ser Pro Glu Leu Asp Cys
125                 130                 135                 140
gag gaa ggg tgg aca cgg tta aag tgt gga gga aac caa aat gaa aga    543
Glu Glu Gly Trp Thr Arg Leu Lys Cys Gly Gly Asn Gln Asn Glu Arg

                145                 150                 155
gcg aag gtg tgc ttt gag aag gct ctg gaa aag aag cca aag aac cca 591
Ala Lys Val Cys Phe Glu Lys Ala Leu Glu Lys Lys Pro Lys Asn Pro
            160                 165                 170
gaa ttc acc tct gga ctg gca ata gca agc tac cgt ctg gac aac tgg 639
Glu Phe Thr Ser Gly Leu Ala Ile Ala Ser Tyr Arg Leu Asp Asn Trp
        175                 180                 185
cca cca tct cag aac gcc att gac cct ctg agg caa gcc att cgg ctg 687
Pro Pro Ser Gln Asn Ala Ile Asp Pro Leu Arg Gln Ala Ile Arg Leu
    190                 195                 200
aat cct gac aac cag tac ctt aaa gtc ctc ctg gct ctg aag ctt cat 735
Asn Pro Asp Asn Gln Tyr Leu Lys Val Leu Leu Ala Leu Lys Leu His
205                 210                 215                 220
aag atg cgt gaa gaa ggt gaa gag gaa ggt gaa gga gag aag tta gtt 783
Lys Met Arg Glu Glu Gly Glu Glu Glu Gly Glu Gly Glu Lys Leu Val
                225                 230                 235
gaa gaa gcc ttg gag aaa gcc cca ggt gta aca gat gtt ctt cgc agt 831
Glu Glu Ala Leu Glu Lys Ala Pro Gly Val Thr Asp Val Leu Arg Ser
            240                 245                 250
gca gcc aag ttt tat cga aga aaa gat gag cca gac aaa gcg att gaa 879
Ala Ala Lys Phe Tyr Arg Arg Lys Asp Glu Pro Asp Lys Ala Ile Glu
        255                 260                 265
ctg ctt aaa aag gct tta gaa tac ata cca aac aat gcc tac ctg cat 927
Leu Leu Lys Lys Ala Leu Glu Tyr Ile Pro Asn Asn Ala Tyr Leu His
    270                 275                 280
tgc caa att ggg tgc tgc tat agg gca aaa gtc ttc caa gta atg aat 975
Cys Gln Ile Gly Cys Cys Tyr Arg Ala Lys Val Phe Gln Val Met Asn
285                 290                 295                 300

cta aga gag aat gga atg tat ggg aaa aga aag tta ctg gaa cta ata 1023
Leu Arg Glu Asn Gly Met Tyr Gly Lys Arg Lys Leu Leu Glu Leu Ile
                305                 310                 315
gga cac gct gtg gct cat ctg aag aaa gct gat gag gcc aat gat aat 1071
Gly His Ala Val Ala His Leu Lys Lys Ala Asp Glu Ala Asn Asp Asn
            320                 325                 330
ctc ttc cgt gtc tgt tcc att ctt gcc agc ctc cat gct cta gca gat 1119
Leu Phe Arg Val Cys Ser Ile Leu Ala Ser Leu His Ala Leu Ala Asp
        335                 340                 345
cag tat gaa gac gca gag tat tac ttc caa aag gaa ttc agt aaa gag 1167
Gln Tyr Glu Asp Ala Glu Tyr Tyr Phe Gln Lys Glu Phe Ser Lys Glu
    350                 355                 360
ctt act cct gta gcg aaa caa ctg ctc cat ctg cgg tat ggc aac ttt 1215
Leu Thr Pro Val Ala Lys Gln Leu Leu His Leu Arg Tyr Gly Asn Phe
365                 370                 375                 380
cag ctg tac caa atg aag tgt gaa gac aag gcc atc cac cac ttt ata 1263
Gln Leu Tyr Gln Met Lys Cys Glu Asp Lys Ala Ile His His Phe Ile
                385                 390                 395
gag ggt gta aaa ata aac cag aaa tca agg gag aaa gaa aag atg aaa 1311
Glu Gly Val Lys Ile Asn Gln Lys Ser Arg Glu Lys Glu Lys Met Lys
            400                 405                 410
gac aaa ctg caa aaa att gcc aaa atg cga ctt tct aaa aat gga gca 1359
Asp Lys Leu Gln Lys Ile Ala Lys Met Arg Leu Ser Lys Asn Gly Ala
        415                 420                 425
gat tct gag gct ttg cat gtc ttg gca ttc ctt cag gag ctg aat gaa 1407
Asp Ser Glu Ala Leu His Val Leu Ala Phe Leu Gln Glu Leu Asn Glu
    430                 435                 440
aaa atg caa caa gca gat gaa gac tct gag agg ggt ttg gag tct gga 1455

Lys Met Gln Gln Ala Asp Glu Asp Ser Glu Arg Gly Leu Glu Ser Gly
445                 450                 455                 460
agc ctc atc cct tca gca tca agc tgg aat ggg gaa tga agaatagaga    1504
Ser Leu Ile Pro Ser Ala Ser Ser Trp Asn Gly Glu
                465                 470
tgtggtgccc actaggctac tgctgaaagg gagctgaaat tcctccacca agttggtatt 1564
caaaatatgt aatgactggt atggcaaaag attggactaa gacactggcc ataccactgg 1624
acagggttat gttaacacct gaattgctgg gtcttgagag agcccaagga gttctgggag 1684
agggaccaga ttggggggta ggtccacggg cttggtgata gaattatttc tcgattgact 1744
tcttgagtgc caatttgaac tgtaaccatt tgcttagtca cctttagtgg agtaatccta 1804
ctgggcttgt ttctatattt atataaagca gccaaatcct tcatgtaata ttgaagtcca 1864
tttttgcaat gttgttccat acttggagtc attttgcagc ccatagaggt tagtcctgca 1924
tagccagtaa tgtgctaagt tcatccaaaa gctggcggac caaagtctaa atagggctca 1984
gtatccccct atcgcttatc tctgcctcct tcctcctcct tcccagtcta tcatcaacct 2044
tgagtattct acacatgtga attcaagtgc ctgattaatt gaggtggcaa catagtttga 2104
gacgagggca gagaacagga agatacatag ctagaagcga cgggtacaaa aagcaatgtg 2164
tacaagaaga ctttcagcaa gtatacagag agttcaccct cctcatagtc ataatgtagc 2224
aagtaaagaa tgagaattgg gttcttctac aatacaacta gaaaccaaca attaatggta 2284
tttctttaaa acctgtgtga aaaaataaat gttgtccacc agtagggata ggggaaaagt 2344
aaccaaaaga gagaaagaga aaggaatgct ggtttatctt tgtagattgt aatcgaatgg 2404
agaaatttgc agtattttag ccactattag gaattttttt tttttgtaaa atgaagactg 2464
aactctgttc aaatgctttc atgaacctgg tttgagacgg taggaaagca acaaaacgtg 2524
ggaacctggt gactaagggc ctggtgcaag gacttgggaa atgtcattga taataaa    2581

<210> 2
<211> 472
<212> PRT

<213> Homo sapiens

<400> 2
Met Ser Glu Asn Asn Lys Asn Ser Leu Glu Ser Ser Leu Arg Gln Leu
1               5                   10                  15
Lys Cys His Phe Thr Trp Asn Leu Met Glu Gly Glu Asn Ser Leu Asp
            20                  25                  30
Asp Phe Glu Asp Lys Val Phe Tyr Arg Thr Glu Phe Gln Asn Arg Glu
        35                  40                  45
Phe Lys Ala Thr Met Cys Asn Leu Leu Ala Tyr Leu Lys His Leu Lys
    50                  55                  60
Gly Gln Asn Glu Ala Ala Leu Glu Cys Leu Arg Lys Ala Glu Glu Leu
65                  70                  75                  80
Ile Gln Gln Glu His Ala Asp Gln Ala Glu Ile Arg Ser Leu Val Thr
                85                  90                  95
Trp Gly Asn Tyr Ala Trp Val Tyr Tyr His Met Gly Arg Leu Ser Asp
            100                 105                 110
Val Gln Ile Tyr Val Asp Lys Val Lys His Val Cys Glu Lys Phe Ser
        115                 120                 125
Ser Pro Tyr Arg Ile Glu Ser Pro Glu Leu Asp Cys Glu Glu Gly Trp
    130                 135                 140
Thr Arg Leu Lys Cys Gly Gly Asn Gln Asn Glu Arg Ala Lys Val Cys
145                 150                 155                 160
Phe Glu Lys Ala Leu Glu Lys Lys Pro Lys Asn Pro Glu Phe Thr Ser
                165                 170                 175
Gly Leu Ala Ile Ala Ser Tyr Arg Leu Asp Asn Trp Pro Pro Ser Gln
            180                 185                 190
Asn Ala Ile Asp Pro Leu Arg Gln Ala Ile Arg Leu Asn Pro Asp Asn

        195                 200                 205
Gln Tyr Leu Lys Val Leu Leu Ala Leu Lys Leu His Lys Met Arg Glu
    210                 215                 220
Glu Gly Glu Glu Glu Gly Glu Gly Glu Lys Leu Val Glu Glu Ala Leu
225                 230                 235                 240
Glu Lys Ala Pro Gly Val Thr Asp Val Leu Arg Ser Ala Ala Lys Phe
                245                 250                 255
Tyr Arg Arg Lys Asp Glu Pro Asp Lys Ala Ile Glu Leu Leu Lys Lys
            260                 265                 270
Ala Leu Glu Tyr Ile Pro Asn Asn Ala Tyr Leu His Cys Gln Ile Gly
        275                 280                 285
Cys Cys Tyr Arg Ala Lys Val Phe Gln Val Met Asn Leu Arg Glu Asn
    290                 295                 300
Gly Met Tyr Gly Lys Arg Lys Leu Leu Glu Leu Ile Gly His Ala Val
305                 310                 315                 320
Ala His Leu Lys Lys Ala Asp Glu Ala Asn Asp Asn Leu Phe Arg Val
                325                 330                 335
Cys Ser Ile Leu Ala Ser Leu His Ala Leu Ala Asp Gln Tyr Glu Asp
            340                 345                 350
Ala Glu Tyr Tyr Phe Gln Lys Glu Phe Ser Lys Glu Leu Thr Pro Val
        355                 360                 365
Ala Lys Gln Leu Leu His Leu Arg Tyr Gly Asn Phe Gln Leu Tyr Gln
    370                 375                 380
Met Lys Cys Glu Asp Lys Ala Ile His His Phe Ile Glu Gly Val Lys
385                 390                 395                 400
Ile Asn Gln Lys Ser Arg Glu Lys Glu Lys Met Lys Asp Lys Leu Gln
                405                 410                 415
Lys Ile Ala Lys Met Arg Leu Ser Lys Asn Gly Ala Asp Ser Glu Ala

420                 425                 430

Leu His Val Leu Ala Phe Leu Gln Glu Leu Asn Glu Lys Met Gln Gln

        435                 440                 445

Ala Asp Glu Asp Ser Glu Arg Gly Leu Glu Ser Gly Ser Leu Ile Pro

    450                 455                 460

Ser Ala Ser Ser Trp Asn Gly Glu

465                 470

<210> 3

<211> 755

<212> DNA

<213> Homo sapiens

<220>

<221> CDS

<222> (53)..(595)

<400> 3

tcctcgtggg ccctgacctt ctctctgaga gccgggcaga ggctccggag cc atg cag 58

                                                          Met Gln

                                                          1

gcc gaa ggc cag ggc aca ggg ggt tcg acg ggc gat gct gat ggc cca    106

Ala Glu Gly Gln Gly Thr Gly Gly Ser Thr Gly Asp Ala Asp Gly Pro

            5                   10                  15

gga ggc cct ggc att cct gat ggc cca ggg ggc aat gct ggc ggc cca    154

Gly Gly Pro Gly Ile Pro Asp Gly Pro Gly Gly Asn Ala Gly Gly Pro

    20                  25                  30

gga gag gcg ggt gcc acg ggc ggc aga ggt ccc cgg ggc gca ggg gca 202
Gly Glu Ala Gly Ala Thr Gly Gly Arg Gly Pro Arg Gly Ala Gly Ala
35                  40                  45                  50
gca agg gcc tcg ggg ccg aga gga ggc gcc ccg cgg ggt ccg cat ggc 250
Ala Arg Ala Ser Gly Pro Arg Gly Gly Ala Pro Arg Gly Pro His Gly
                55                  60                  65
ggt gcc gct tct gcg cag gat gga agg tgc ccc tgc ggg gcc agg agg 298
Gly Ala Ala Ser Ala Gln Asp Gly Arg Cys Pro Cys Gly Ala Arg Arg
            70                  75                  80
ccg gac agc cgc ctg ctt cag ttg cac atc acg atg cct ttc tcg tcg 346
Pro Asp Ser Arg Leu Leu Gln Leu His Ile Thr Met Pro Phe Ser Ser
        85                  90                  95
ccc atg gaa gcg gag ctg gtc cgc agg atc ctg tcc cgg gat gcc gca 394
Pro Met Glu Ala Glu Leu Val Arg Arg Ile Leu Ser Arg Asp Ala Ala
    100                 105                 110
cct ctc ccc cga cca ggg gcg gtt ctg aag gac ttc acc gtg tcc ggc 442
Pro Leu Pro Arg Pro Gly Ala Val Leu Lys Asp Phe Thr Val Ser Gly
115                 120                 125                 130
aac cta ctg ttt atc cga ctg act gct gca gac cac cgc caa ctg cag 490
Asn Leu Leu Phe Ile Arg Leu Thr Ala Ala Asp His Arg Gln Leu Gln
                135                 140                 145
ctc tcc atc agc tcc tgt ctc cag cag ctt tcc ctg ttg atg tgg atc 538
Leu Ser Ile Ser Ser Cys Leu Gln Gln Leu Ser Leu Leu Met Trp Ile
            150                 155                 160
acg cag tgc ttt ctg ccc gtg ttt ttg gct cag gct ccc tca ggg cag 586
Thr Gln Cys Phe Leu Pro Val Phe Leu Ala Gln Ala Pro Ser Gly Gln
        165                 170                 175
agg cgc taa gcccagcctg gcgccccttc ctaggtcatg cctcctcccc 635

Arg Arg
    180
tagggaatgg tcccagcacg agtggccagt tcattgtggg ggcctgattg tttgtcgctg 695
gaggaggacg gcttacatgt ttgtttctgt agaaaataaa gctgagctac gaaaaaaaaa 755

<210> 4
<211> 180
<212> PRT
<213> Homo sapiens

<400> 4
Met Gln Ala Glu Gly Gln Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30
Gly Pro Gly Glu Ala Gly Ala Thr Gly Gly Arg Gly Pro Arg Gly Ala
        35                  40                  45
Gly Ala Ala Arg Ala Ser Gly Pro Arg Gly Gly Ala Pro Arg Gly Pro
    50                  55                  60
His Gly Gly Ala Ala Ser Ala Gln Asp Gly Arg Cys Pro Cys Gly Ala
65                  70                  75                  80
Arg Arg Pro Asp Ser Arg Leu Leu Gln Leu His Ile Thr Met Pro Phe
                85                  90                  95
Ser Ser Pro Met Glu Ala Glu Leu Val Arg Arg Ile Leu Ser Arg Asp
            100                 105                 110
Ala Ala Pro Leu Pro Arg Pro Gly Ala Val Leu Lys Asp Phe Thr Val
        115                 120                 125

Ser Gly Asn Leu Leu Phe Ile Arg Leu Thr Ala Ala Asp His Arg Gln
    130                 135                 140
Leu Gln Leu Ser Ile Ser Ser Cys Leu Gln Gln Leu Ser Leu Leu Met
145                 150                 155                 160
Trp Ile Thr Gln Cys Phe Leu Pro Val Phe Leu Ala Gln Ala Pro Ser
                165                 170                 175
Gly Gln Arg Arg
            180

<210> 5
<211> 1004
<212> DNA
<213> Homo sapiens

<220>
<221> CDS
<222> (201)..(332)

<400> 5
cgccaattta gggtctccgg tatctcccgc tgagctgctc tgttcccggc ttagaggacc 60
aggagaaggg ggagctggag gctggagcct gtaacaccgt ggctcgtctc actctggatg 120
gtggtggcaa cagagatggc agcgcagctg gagtgttagg agggcggcct gagcggtagg 180
agtggggctg gagcagtaag atg gcg gcc aga gcg gtt ttt ctg gca ttg tct 233
                     Met Ala Ala Arg Ala Val Phe Leu Ala Leu Ser
                     1               5                   10
gcc cag ctg ctc caa gcc agg ctg atg aag gag gag tcc cct gtg gtg   281
Ala Gln Leu Leu Gln Ala Arg Leu Met Lys Glu Glu Ser Pro Val Val

15                  20                  25

agc tgg agg ttg gag cct gaa gac ggc aca gct ctg tgc ttc atc ttc    329

Ser Trp Arg Leu Glu Pro Glu Asp Gly Thr Ala Leu Cys Phe Ile Phe

        30                  35                  40

tga ggttgtggca gccacggtga tggagacggc agctcaacag gagcaatagg         382

aggagatgga gtttcactgt gtcagccagg atggtctcga tctcctgacc tcgtgatccg 442

cccgccttgg ccttccaaag tgccgagatt acagcgatgt gcattttgta agcactttgg 502

agccactatc aaatgctgtg aagagaaatg tacccagatg tatcattatc cttgtgctgc 562

aggagccggc tcctttcagg atttcagtca catcttcctg ctttgtccag aacacattga 622

ccaagctcct gaaagatgta agtttactac gcatagactt ttaaacttca accaatgtat 682

ttactgaaaa taacaaatgt tgtaaattcc ctgagtgtta ttctacttgt attaaaaggt 742

aataatacat aatcattaaa atctgaggga tcattgccag agattgttgg ggagggaaat 802

gttatcaacg gtttcattga aattaaatcc aaaaagttat ttcctcagaa aaatcaaata 862

aagtttgcat gttttttatt cttaaaacat tttaaaaacc actgtagaat gatgtaaata 922

gggactgtgc agtatttctg acatatacta taaaattatt aaaaagtcaa tcagtattca 982

acatctttta cactaaaaag cc                                          1004

<210> 6

<211> 43

<212> PRT

<213> Homo sapiens

<400> 6

Met Ala Ala Arg Ala Val Phe Leu Ala Leu Ser Ala Gln Leu Leu Gln

1               5                   10                  15

Ala Arg Leu Met Lys Glu Glu Ser Pro Val Val Ser Trp Arg Leu Glu

            20                  25                  30

Pro Glu Asp Gly Thr Ala Leu Cys Phe Ile Phe

        35                  40

<210> 7

<211> 4193

<212> DNA

<213> Homo sapiens

<220>

<221> CDS

<222> (101)..(3523)

<400> 7
gtggagttcg ctgcggctgt tgggggccac ctgtcttttc gcttgtgccc ctctttctag 60
tgtcgcgctc gagtcccgac gggccgctcc aagcctcgac atg tcg tac aac tac 115
gtg gta acg gcc cag aag ccc acc gcc gtg aac ggc tgc gtg acc gga 163
cac ttt act tcg gcc gaa gac tta aac ctg ttg att gcc aaa aac acg 211
aga tta gag atc tat gtg gtc acc gcc gag ggg ctt cgg ccc gtc aaa 259
gag gtg ggc atg tat ggg aag att gcg gtc atg gag ctt ttc agg ccc 307
aag ggg gag agc aag gac ctg ctg ttt atc ttg aca gcg aag tac aat 355
gcc tgc atc ctg gag tat aaa cag agt ggc gag agc att gac atc att 403
acg cga gcc cat ggc aat gtc cag gac cgc att ggc cgc ccc tca gag 451
acc ggc att att ggc atc att gac cct gag tgc cgg atg att ggc ctg 499
cgt ctc tat gat ggc ctt ttc aag gtt att cca cta gat cgc gat aat 547
aaa gaa ctc aag gcc ttc aac atc cgc ctg gag gag ctg cat gtc att 595
gat gtc aag ttc cta tat ggt tgc caa gca cct act att tgc ttt gtc 643
tac cag gac cct cag ggg cgg cac gta aaa acc tat gag gtg tct ctc 691
cga gaa aag gaa ttc aat aag ggc cct tgg aaa cag gaa aat gtc gaa 739
gct gaa gct tcc atg gtg atc gca gtc cca gag ccc ttt ggg ggg gcc 787
atc atc att gga cag gag tca atc acc tat cac aat ggt gac aaa tac 835
ctg gct att gcc cct cct atc atc aag caa agc acg att gtg tgc cac 883
aat cga gtg gac cct aat ggc tca aga tac ctg ctg gga gac atg gaa 931
ggc cgg ctc ttc atg ctg ctt ttg gag aag gag gaa cag atg gat ggc 979
acc gtc act ctc aag gat ctc cgt gta gaa ctc ctt gga gag acc tct 1027
att gct gag tgc ttg aca tac ctt gat aat ggt gtt gtg ttt gtc ggg 1075
tct cgc ctg ggt gac tcc cag ctt gtg aag ctc aac gtt gac agt aat 1123
gaa caa ggc tcc tat gta gtg gcc atg gaa acc ttt acc aac tta gga 1171
ccc att gtc gat atg tgc gtg gtg gac ctg gag agg cag ggg cag ggg 1219
cag ctg gtc act tgc tct ggg gct ttc aag gaa ggt tct ttg cgg atc 1267
atc cgg aat gga att gga atc cac gag cat gcc agc att gac tta cca 1315
ggc atc aaa gga tta tgg cca ctg cgg tct gac cct aat cgt gag act 1363
gat gac act ttg gtg ctc tct ttt gtg ggc cag aca aga gtt ctc atg 1411
tta aat gga gag gag gta gaa gaa acc gaa ctg atg ggt ttc gtg gat 1459
gat cag cag act ttc ttc tgt ggc aac gtg gct cat cag cag ctt atc 1507
cag atc act tca gca tcg gtg agg ttg gtc tct caa gaa ccc aaa gct 1555
ctg gtc agt gaa tgg aag gag cct cag gcc aag aac atc agt gtg gcc 1603
tcc tgc aat agc agc cag gtg gtg gtg gct gta ggc agg gcc ctc tac 1651
tat ctg cag atc cat cct cag gag ctc cgg cag atc agc cac aca gag 1699
atg gaa cat gaa gtg gct tgc ttg gac atc acc cca tta gga gac agc 1747
aat gga ctg tcc cct ctt tgt gcc att ggc ctc tgg acg gac atc tcg 1795
gct cgt atc ttg aag ttg ccc tct ttt gaa cta ctg cac aag gag atg 1843
ctg ggt gga gag atc att cct cgc tcc atc ctg atg acc acc ttt gag 1891
agt agc cat tac ctc ctt tgt gcc ttg gga gat gga gcg ctt ttc tac 1939
ttt ggg ctc aac att gag aca ggt ctg ttg agc gac cgt aag aag gtg 1987
act ttg ggc acc cag ccc acc gta ttg agg act ttt cgt tct ctt tct 2035
acc acc aac gtc ttt gct tgt tct gac cgc ccc act gtc atc tat agc 2083
agc aac cac aaa ttg gtc ttc tca aat gtc aac ctc aag gaa gtg aac 2131
tac atg tgt ccc ctc aat tca gat ggc tat cct gac agc ctg gcg ctg 2179
gcc aac aat agc acc ctc acc att ggc acc atc gat gag atc cag aag 2227
ctg cac att cgc aca gtt ccc ctc tat gag tct cca agg aag atc tgc 2275
tac cag gaa gtg tcc cag tgt ttc ggg gtc ctc tcc agc cgc att gaa 2323
gtc caa gac acg agt ggg ggc acg aca gcc ttg agg ccc agc gct agc 2371
acc cag gct ctg tcc agc agt gta agc tcc agc aag ctg ttc tcc agc 2419
agc act gct cct cat gag acc tcc ttt gga gaa gag gtg gag gtg cac 2467
aac cta ctt atc att gac caa cac acc ttt gaa gtg ctt cat gcc cac 2515
cag ttt ctg cag aat gaa tat gcc ctc agt ctg gtt tcc tgc aag ctg 2563
ggc aaa gac ccc aac act tac ttc att gtg ggc aca gca atg gtg tat 2611
cct gaa gag gca gag ccc aag cag ggt cgc att gtg gtc ttt cag tat 2659
tcg gat gga aaa cta cag act gtg gct gaa aag gaa gtg aaa ggg gcc 2707
gtg tac tct atg gtg gaa ttt aac ggg aag ctg tta gcc agc atc aat 2755
agc acg gtg cgg ctc tat gag tgg aca aca gag aag gag ctg cgc act 2803
gag tgc aac cac tac aac aac atc atg gcc ctc tac ctg aag acc aag 2851
ggc gac ttc atc ctg gtg ggc gac ctt atg cgc tca gtg ctg ctg ctt 2899
gcc tac aag ccc atg gaa gga aac ttt gaa gag att gct cga gac ttt 2947
aat ccc aac tgg atg agt gct gtg gaa atc ttg gat gat gac aat ttt 2995
ctg ggg gct gaa aat gcc ttt aac ttg ttt gtg tgt caa aag gat agc     3043
gct gcc acc act gac gag gag cgg cag cac ctc cag gag gtt ggt ctt     3091
ttc cac ctg ggc gag ttt gtc aat gtc ttt tgc cac ggc tct ctg gta     3139
atg cag aat ctg ggt gag act tcc acc ccc aca caa ggc tcg gtg ctc     3187
ttc ggc acg gtc aac ggc atg ata ggg ctg gtg acc tca ctg tca gag     3235
agc tgg tac aac ctc ctg ctg gac atg cag aat cga ctc aat aaa gtc     3283
atc aaa agt gtg ggg aag atc gag cac tcc ttc tgg aga tcc ttt cac     3331
acc gag cgg aag aca gaa cca gcc aca ggt ttc atc gac ggt gac ttg     3379
att gag agt ttc ctg gat att agc cgc ccc aag atg cag gag gtg gtg     3427
gca aac cta cag tat gac gat ggc agc ggt atg aag cga gag gcc act    3475
gca gac gac ctc atc aag gtt gtg gag gag cta act cgg atc cat tag    3523
ccaagggcag ggggccccct ttctgaccct ccccaaaggc tttgccctgc tgccctcccc 3583
ctcctctcca ccatcgtctt cttggccatg ggaggccttt ccctaagcca gctgccccca 3643
gagccacagt tcccctatgt ggaagtgggg cgggcttcat agagacttgg gaatgagctg 3703
aaggtgaaac attttctccc tggattttta ccagtctcac atgattccag ccatcacctt 3763
agaccaccaa gccttgattg gtgttgccag ttgtcctcct tccggggaag gattttgcag 3823
ttctttggct gaaaggaagc tgtgcgtgtg tgtgtgtgta tgtgtgtgtg tgtatgtgta 3883
tctcacactc atgcaatgtc ctctttttat ttagattggc agtgtaggga gttgtgggta 3943
gtggggaaga gggttaggag ggtttcattg tctgtgaagt gagaccttcc ttttactttt 4003
cttctattgc ctctgagagc atcagcctag aggcctgact gccaagccat gggtagcctg 4063
ggtgtaaaac ctggagatgg tggatgatcc ccacgccaca gcccttttgt ctctgcaaac 4123
tgccttcttc ggaaagaaga aggtgggagg atgtgaattg ttagtttctg agttttacca 4183
aataaagtag                                                        4193

<210> 8

<211> 1140

<212> PRT

<213> Homo sapiens

<400> 8

<210> 9

<211> 2269

<212> DNA

<213> Homo sapiens

<220>

<221> CDS

<222> (292)..(1701)

<400> 9

ccgtttcctc ccctcccctc cactcggccg tccctccttc ctcctccctc ctccctcctc 60

ctcccgctcc tgaagagcgc gccgcgtggg ggacggcccg gttacttcct ccagagactg 120

acgagtgcgg tgtcgctcca gctcagagct cccggagccg cccggccagc gtccggcctc 180

cctgatcgtc tctggccggc gccctcgccc tcgcccggcg cgcaccgagc agccgcgggc 240

gccgagcagc caccgtcccg accaagcgcc ggccctgccc gcagcggcag g atg aat 297

                                                        Met Asn

                                                        1

gat ttc gga atc aag aat atg gac cag gta gcc cct gtg gct aac agt 345

Asp Phe Gly Ile Lys Asn Met Asp Gln Val Ala Pro Val Ala Asn Ser

        5                   10                  15

tac aga ggg aca ctc aag cgc cag cca gcc ttt gac acc ttt gat ggg 393

Tyr Arg Gly Thr Leu Lys Arg Gln Pro Ala Phe Asp Thr Phe Asp Gly

    20                  25                  30

tcc ctg ttt gct gtt ttt cct tct cta aat gaa gag caa aca ctg caa 441
Ser Leu Phe Ala Val Phe Pro Ser Leu Asn Glu Glu Gln Thr Leu Gln
35                  40                  45                  50
gaa gtg cca aca ggc ttg gat tcc att tct cat gac tcc gcc aac tgt 489
Glu Val Pro Thr Gly Leu Asp Ser Ile Ser His Asp Ser Ala Asn Cys
                55                  60                  65
gaa ttg cct ttg tta acc ccg tgc agc aag gct gtg atg agt caa gcc 537
Glu Leu Pro Leu Leu Thr Pro Cys Ser Lys Ala Val Met Ser Gln Ala
            70                  75                  80
tta aaa gct acc ttc agt ggc ttc aaa aag gaa cag cgg cgc ctg ggc 585
Leu Lys Ala Thr Phe Ser Gly Phe Lys Lys Glu Gln Arg Arg Leu Gly
        85                  90                  95
att cca aag aac ccc tgg ctg tgg agt gag caa cag gta tgc cag tgg 633
Ile Pro Lys Asn Pro Trp Leu Trp Ser Glu Gln Gln Val Cys Gln Trp
    100                 105                 110
ctt ctc tgg gcc acc aat gag ttc agt ctg gtg aac gtg aat ctg cag 681
Leu Leu Trp Ala Thr Asn Glu Phe Ser Leu Val Asn Val Asn Leu Gln
115                 120                 125                 130
agg ttc ggc atg aat ggc cag atg ctg tgt aac ctt ggc aag gaa cgc 729
Arg Phe Gly Met Asn Gly Gln Met Leu Cys Asn Leu Gly Lys Glu Arg
                135                 140                 145
ttt ctg gag ctg gca cct gac ttt gtg ggt gac att ctc tgg gaa cat 777
Phe Leu Glu Leu Ala Pro Asp Phe Val Gly Asp Ile Leu Trp Glu His
            150                 155                 160
ctg gag caa atg atc aaa gaa aac caa gaa aag aca gaa gat caa tat 825
Leu Glu Gln Met Ile Lys Glu Asn Gln Glu Lys Thr Glu Asp Gln Tyr
        165                 170                 175
gaa gaa aat tca cac ctc acc tcc gtt cct cat tgg att aac agc aat 873

Glu Glu Asn Ser His Leu Thr Ser Val Pro His Trp Ile Asn Ser Asn
    180                 185                 190
aca tta ggt ttt ggc aca gag cag gcg ccc tat gga atg cag aca cag 921
Thr Leu Gly Phe Gly Thr Glu Gln Ala Pro Tyr Gly Met Gln Thr Gln
195                 200                 205                 210
aat tac ccc aaa ggc ggc ctc ctg gac agc atg tgt ccg gcc tcc aca 969
Asn Tyr Pro Lys Gly Gly Leu Leu Asp Ser Met Cys Pro Ala Ser Thr
                215                 220                 225
ccc agc gta ctc agc tct gag cag gag ttt cag atg ttc ccc aag tct 1017
Pro Ser Val Leu Ser Ser Glu Gln Glu Phe Gln Met Phe Pro Lys Ser
            230                 235                 240
cgg ctc agc tcc gtc agc gtc acc tac tgc tct gtc agt cag gac ttc 1065
Arg Leu Ser Ser Val Ser Val Thr Tyr Cys Ser Val Ser Gln Asp Phe
        245                 250                 255
cca ggc agc aac ttg aat ttg ctc acc aac aat tct ggg act ccc aaa 1113
Pro Gly Ser Asn Leu Asn Leu Leu Thr Asn Asn Ser Gly Thr Pro Lys
    260                 265                 270
gac cac gac tcc cct gag aac ggt gcg gac agc ttc gag agc tca gac 1161
Asp His Asp Ser Pro Glu Asn Gly Ala Asp Ser Phe Glu Ser Ser Asp
275                 280                 285                 290
tcc ctc ctc cag tcc tgg aac agc cag tcg tcc ttg ctg gat gtg caa 1209
Ser Leu Leu Gln Ser Trp Asn Ser Gln Ser Ser Leu Leu Asp Val Gln
                295                 300                 305
cgg gtt cct tcc ttc gag agc ttc gaa gat gac tgc agc cag tct ctc 1257
Arg Val Pro Ser Phe Glu Ser Phe Glu Asp Asp Cys Ser Gln Ser Leu
            310                 315                 320
tgc ctc aat aag cca acc atg tct ttc aag gat tac atc caa gag agg 1305
Cys Leu Asn Lys Pro Thr Met Ser Phe Lys Asp Tyr Ile Gln Glu Arg

        325                 330                 335

agt gac cca gtg gag caa ggc aaa cca gtt ata cct gca gct gtg ctg 1353

Ser Asp Pro Val Glu Gln Gly Lys Pro Val Ile Pro Ala Ala Val Leu

    340                 345                 350

gcc ggc ttc aca gga agt gga cct att cag ctg tgg cag ttt ctc ctg 1401

Ala Gly Phe Thr Gly Ser Gly Pro Ile Gln Leu Trp Gln Phe Leu Leu

355                 360                 365                 370

gag ctg cta tca gac aaa tcc tgc cag tca ttc atc agc tgg act gga 1449

Glu Leu Leu Ser Asp Lys Ser Cys Gln Ser Phe Ile Ser Trp Thr Gly

                375                 380                 385

gac gga tgg gag ttt aag ctc gcc gac ccc gat gag gtg gcc cgc cgg 1497

Asp Gly Trp Glu Phe Lys Leu Ala Asp Pro Asp Glu Val Ala Arg Arg

            390                 395                 400

tgg gga aag agg aaa aat aag ccc aag atg aac tac gag aag ctg agc 1545

Trp Gly Lys Arg Lys Asn Lys Pro Lys Met Asn Tyr Glu Lys Leu Ser

        405                 410                 415

cgg ggc tta cgc tac tat tac gac aag aac atc atc cac aag acg tcg 1593

Arg Gly Leu Arg Tyr Tyr Tyr Asp Lys Asn Ile Ile His Lys Thr Ser

    420                 425                 430

ggg aag cgc tac gtg tac cgc ttc gtg tgc gac ctc cag aac ttg ctg 1641

Gly Lys Arg Tyr Val Tyr Arg Phe Val Cys Asp Leu Gln Asn Leu Leu

435                 440                 445                 450

ggg ttc acg ccc gag gaa ctg cac gcc atc ctg ggc gtc cag ccc gac 1689

Gly Phe Thr Pro Glu Glu Leu His Ala Ile Leu Gly Val Gln Pro Asp

                455                 460                 465

acg gag gac tga ggtcgccggg accaccctga gccggcccca ggctcgtgga 1741

Thr Glu Asp

470

ctgagtggga agcccatcct gaccagctgc ctccgaggac ccaggaaagg caggattgaa 1801

aatgtccagg aaagtggcca agaagcagtg gccttattgc atcccaaacc acgcctcttg 1861

accaggctgc ctcccttgtg gcagcaacgg cacagctaat tctactcaca gtgcttttaa 1921

gtgaaaatgg tcgagaaaga ggcaccggga agccgtcctg gcgcctggca gtccgtggga 1981

cgggatggtt ctggctgttt gagattctca aaggagcgag catgtcgtgg acacacacag 2041

actattttta gatttctttt tgccttttgc aaccaggaac agcaaatgca aaaactcttt 2101

gagagggtag gagggtggga aggaaacaac catgtcattt cagaagttag tttgtatata 2161

ttataataat cttataattg ttctcagaat cccttaacag ttgtatttaa cagaaattgt 2221

atattgtaat ttaaaataat tatataactg tatttgaaat aagaattc 2269

<210> 10

<211> 469

<212> PRT

<213> Homo sapiens

<400> 10

Met Asn Asp Phe Gly Ile Lys Asn Met Asp Gln Val Ala Pro Val Ala
1               5                   10                  15

Asn Ser Tyr Arg Gly Thr Leu Lys Arg Gln Pro Ala Phe Asp Thr Phe
            20                  25                  30

Asp Gly Ser Leu Phe Ala Val Phe Pro Ser Leu Asn Glu Glu Gln Thr
        35                  40                  45

Leu Gln Glu Val Pro Thr Gly Leu Asp Ser Ile Ser His Asp Ser Ala
    50                  55                  60

Asn Cys Glu Leu Pro Leu Leu Thr Pro Cys Ser Lys Ala Val Met Ser
65                  70                  75                  80

Gln Ala Leu Lys Ala Thr Phe Ser Gly Phe Lys Lys Glu Gln Arg Arg

                85                  90                  95
Leu Gly Ile Pro Lys Asn Pro Trp Leu Trp Ser Glu Gln Gln Val Cys
            100                 105                 110
Gln Trp Leu Leu Trp Ala Thr Asn Glu Phe Ser Leu Val Asn Val Asn
        115                 120                 125
Leu Gln Arg Phe Gly Met Asn Gly Gln Met Leu Cys Asn Leu Gly Lys
    130                 135                 140
Glu Arg Phe Leu Glu Leu Ala Pro Asp Phe Val Gly Asp Ile Leu Trp
145                 150                 155                 160
Glu His Leu Glu Gln Met Ile Lys Glu Asn Gln Glu Lys Thr Glu Asp
                165                 170                 175
Gln Tyr Glu Glu Asn Ser His Leu Thr Ser Val Pro His Trp Ile Asn
            180                 185                 190
Ser Asn Thr Leu Gly Phe Gly Thr Glu Gln Ala Pro Tyr Gly Met Gln
        195                 200                 205
Thr Gln Asn Tyr Pro Lys Gly Gly Leu Leu Asp Ser Met Cys Pro Ala
    210                 215                 220
Ser Thr Pro Ser Val Leu Ser Ser Glu Gln Glu Phe Gln Met Phe Pro
225                 230                 235                 240
Lys Ser Arg Leu Ser Ser Val Ser Val Thr Tyr Cys Ser Val Ser Gln
                245                 250                 255
Asp Phe Pro Gly Ser Asn Leu Asn Leu Leu Thr Asn Asn Ser Gly Thr
            260                 265                 270
Pro Lys Asp His Asp Ser Pro Glu Asn Gly Ala Asp Ser Phe Glu Ser
        275                 280                 285
Ser Asp Ser Leu Leu Gln Ser Trp Asn Ser Gln Ser Ser Leu Leu Asp
    290                 295                 300
Val Gln Arg Val Pro Ser Phe Glu Ser Phe Glu Asp Asp Cys Ser Gln

305                 310                 315                 320

Ser Leu Cys Leu Asn Lys Pro Thr Met Ser Phe Lys Asp Tyr Ile Gln

                325                 330                 335

Glu Arg Ser Asp Pro Val Glu Gln Gly Lys Pro Val Ile Pro Ala Ala

            340                 345                 350

Val Leu Ala Gly Phe Thr Gly Ser Gly Pro Ile Gln Leu Trp Gln Phe

        355                 360                 365

Leu Leu Glu Leu Leu Ser Asp Lys Ser Cys Gln Ser Phe Ile Ser Trp

    370                 375                 380

Thr Gly Asp Gly Trp Glu Phe Lys Leu Ala Asp Pro Asp Glu Val Ala

385                 390                 395                 400

Arg Arg Trp Gly Lys Arg Lys Asn Lys Pro Lys Met Asn Tyr Glu Lys

                405                 410                 415

Leu Ser Arg Gly Leu Arg Tyr Tyr Tyr Asp Lys Asn Ile Ile His Lys

            420                 425                 430

Thr Ser Gly Lys Arg Tyr Val Tyr Arg Phe Val Cys Asp Leu Gln Asn

        435                 440                 445

Leu Leu Gly Phe Thr Pro Glu Glu Leu His Ala Ile Leu Gly Val Gln

    450                 455                 460

Pro Asp Thr Glu Asp

465

<210> 11

<211> 1533

<212> DNA

<213> Homo sapiens

<220>

<221> CDS

<222> (1)..(1533)

<400> 11

atg gcg gcg gag ctg gtg gag gcc aaa aac atg gtg atg agt ttt cga    48

Met Ala Ala Glu Leu Val Glu Ala Lys Asn Met Val Met Ser Phe Arg

1               5                   10                  15

gtc tcc gac ctt cag atg ctc ctg ggt ttc gtg ggc cgg agt aag agt    96

Val Ser Asp Leu Gln Met Leu Leu Gly Phe Val Gly Arg Ser Lys Ser

            20                  25                  30

gga ctg aag cac gag ctc gtc acc agg gcc ctc cag ctg gtg cag ttt    144

Gly Leu Lys His Glu Leu Val Thr Arg Ala Leu Gln Leu Val Gln Phe

        35                  40                  45

gac tgt agc cct gag ctg ttc aag aag atc aag gag ctg tac gag acc    192

Asp Cys Ser Pro Glu Leu Phe Lys Lys Ile Lys Glu Leu Tyr Glu Thr

    50                  55                  60

cgc tac gcc aag aag aac tcg gag cct gcc cca cag ccg cac cgg ccc    240

Arg Tyr Ala Lys Lys Asn Ser Glu Pro Ala Pro Gln Pro His Arg Pro

65                  70                  75                  80

ctg gac ccc ctg acc atg cac tcc acc tac gac cgg gcc ggc gct gtg    288

Leu Asp Pro Leu Thr Met His Ser Thr Tyr Asp Arg Ala Gly Ala Val

                85                  90                  95

ccc agg act ccg ctg gca ggc ccc aat att gac tac ccc gtg ctc tac    336

Pro Arg Thr Pro Leu Ala Gly Pro Asn Ile Asp Tyr Pro Val Leu Tyr

            100                 105                 110

gga aag tac tta aac gga ctg gga cgg ttg ccc gcc aag acc ctc aag    384

Gly Lys Tyr Leu Asn Gly Leu Gly Arg Leu Pro Ala Lys Thr Leu Lys

115                 120                 125

cca gaa gtc cgc ctg gtg aag ctg ccg ttc ttt aat atg ctg gat gag     432

Pro Glu Val Arg Leu Val Lys Leu Pro Phe Phe Asn Met Leu Asp Glu

    130                 135                 140

ctg ctg aag ccc acc gaa tta gtc cca cag aac aac gag aag ctt cag     480

Leu Leu Lys Pro Thr Glu Leu Val Pro Gln Asn Asn Glu Lys Leu Gln

145                 150                 155                 160

gag agc ccg tgc atc ttc gca ttg acg cca aga cag gtg gag ttg atc     528

Glu Ser Pro Cys Ile Phe Ala Leu Thr Pro Arg Gln Val Glu Leu Ile

                165                 170                 175

cgg aaa ttc cag gga atg cag ccc gga gtt aaa gcc gtg cag gtc gtc     576

Arg Lys Phe Gln Gly Met Gln Pro Gly Val Lys Ala Val Gln Val Val

            180                 185                 190

ctg aga atc tgt tac tca gac acc agc tgc cct cag gag gac cag tac     624

Leu Arg Ile Cys Tyr Ser Asp Thr Ser Cys Pro Gln Glu Asp Gln Tyr

        195                 200                 205

ccg ccc aac atc gct gtg aag gtc aac cac agc tac tgc tcc gtc ccg     672

Pro Pro Asn Ile Ala Val Lys Val Asn His Ser Tyr Cys Ser Val Pro

    210                 215                 220

ggc tac tac ccc tcc aat aag ccc ggg gtg gag ccc aag agg ccg tgc     720

Gly Tyr Tyr Pro Ser Asn Lys Pro Gly Val Glu Pro Lys Arg Pro Cys

225                 230                 235                 240

cgc ccc atc aac ctc act cac ctc atg tac ctg tcc tcg gcc acc aac     768

Arg Pro Ile Asn Leu Thr His Leu Met Tyr Leu Ser Ser Ala Thr Asn

                245                 250                 255

cgc atc act gtc acc tgg ggg aac tac ggc aag agc tac tcg gtg gcc     816

Arg Ile Thr Val Thr Trp Gly Asn Tyr Gly Lys Ser Tyr Ser Val Ala

            260                 265                 270

ctg tac ctg gtg cgg cag ctg acc tca tcg gag ctg ctg cag agg ctg 864
Leu Tyr Leu Val Arg Gln Leu Thr Ser Ser Glu Leu Leu Gln Arg Leu
        275                 280                 285
aag acc att ggg gta aag cac ccg gag ctg tgc aag gca ctg gtc aag 912
Lys Thr Ile Gly Val Lys His Pro Glu Leu Cys Lys Ala Leu Val Lys
    290                 295                 300
gag aag ctg cgc ctt gat cct gac agc gag atc gcc acc acc ggt gtg 960
Glu Lys Leu Arg Leu Asp Pro Asp Ser Glu Ile Ala Thr Thr Gly Val
305                 310                 315                 320
cgg gtg tcc ctc atc tgt ccg ctg gtg aag atg cgg ctc tcc gtg ccc 1008
Arg Val Ser Leu Ile Cys Pro Leu Val Lys Met Arg Leu Ser Val Pro
                325                 330                 335
tgc cgg gca gaa acc tgc gcc cac ctg cag tgc ttc gac gcc gtc ttc 1056
Cys Arg Ala Glu Thr Cys Ala His Leu Gln Cys Phe Asp Ala Val Phe
            340                 345                 350
tac ctg cag atg aac gag aag aag ccc acc tgg atg tgc ccc gtg tgc 1104
Tyr Leu Gln Met Asn Glu Lys Lys Pro Thr Trp Met Cys Pro Val Cys
        355                 360                 365
gac aag cca gcc ccc tac gac cag ctc atc atc gac ggg ctc ctc tcg 1152
Asp Lys Pro Ala Pro Tyr Asp Gln Leu Ile Ile Asp Gly Leu Leu Ser
    370                 375                 380
aag atc ctg agc gag tgt gag gac gcc gac gag atc gag tac ctg gtg 1200
Lys Ile Leu Ser Glu Cys Glu Asp Ala Asp Glu Ile Glu Tyr Leu Val
385                 390                 395                 400
gac ggc tcg tgg tgc ccg atc cgc gcc gaa aag gag cgc agc tgc agc 1248
Asp Gly Ser Trp Cys Pro Ile Arg Ala Glu Lys Glu Arg Ser Cys Ser
                405                 410                 415
ccg cag ggc gcc atc ctc gtg ctg ggc ccc tcg gac gcc aat ggg ctc 1296

Pro Gln Gly Ala Ile Leu Val Leu Gly Pro Ser Asp Ala Asn Gly Leu
            420                 425                 430
ctg ccc gcc ccc agc gtc aac ggg agc ggt gcc ctg ggc agc acg ggt     1344
Leu Pro Ala Pro Ser Val Asn Gly Ser Gly Ala Leu Gly Ser Thr Gly
        435                 440                 445
ggc ggc ggc ccg gtg ggc agc atg gag aat ggg aag ccg ggc gcc gat     1392
Gly Gly Gly Pro Val Gly Ser Met Glu Asn Gly Lys Pro Gly Ala Asp
    450                 455                 460
gtg gtg gac ctc acg ctg gac agc tca tcg tcc tcg gag gat gag gag     1440
Val Val Asp Leu Thr Leu Asp Ser Ser Ser Ser Ser Glu Asp Glu Glu
465                 470                 475                 480
gag gag gaa gag gag gag gaa gac gag gac gaa gag ggg ccc cgg ccc     1488
Glu Glu Glu Glu Glu Glu Glu Asp Glu Asp Glu Glu Gly Pro Arg Pro
                485                 490                 495
aag cgc cgc tgc ccc ttc cag aag ggc ctg gtg ccg gcc tgc tga         1533
Lys Arg Arg Cys Pro Phe Gln Lys Gly Leu Val Pro Ala Cys
            500                 505                 510

<210> 12
<211> 510
<212> PRT
<213> Homo sapiens

<400> 12
Met Ala Ala Glu Leu Val Glu Ala Lys Asn Met Val Met Ser Phe Arg
  1               5                  10                  15
Val Ser Asp Leu Gln Met Leu Leu Gly Phe Val Gly Arg Ser Lys Ser

20                  25                  30
Gly Leu Lys His Glu Leu Val Thr Arg Ala Leu Gln Leu Val Gln Phe
        35                  40                  45
Asp Cys Ser Pro Glu Leu Phe Lys Lys Ile Lys Glu Leu Tyr Glu Thr
    50                  55                  60
Arg Tyr Ala Lys Lys Asn Ser Glu Pro Ala Pro Gln Pro His Arg Pro
65                  70                  75                  80
Leu Asp Pro Leu Thr Met His Ser Thr Tyr Asp Arg Ala Gly Ala Val
                85                  90                  95
Pro Arg Thr Pro Leu Ala Gly Pro Asn Ile Asp Tyr Pro Val Leu Tyr
            100                 105                 110
Gly Lys Tyr Leu Asn Gly Leu Gly Arg Leu Pro Ala Lys Thr Leu Lys
        115                 120                 125
Pro Glu Val Arg Leu Val Lys Leu Pro Phe Phe Asn Met Leu Asp Glu
    130                 135                 140
Leu Leu Lys Pro Thr Glu Leu Val Pro Gln Asn Asn Glu Lys Leu Gln
145                 150                 155                 160
Glu Ser Pro Cys Ile Phe Ala Leu Thr Pro Arg Gln Val Glu Leu Ile
                165                 170                 175
Arg Lys Phe Gln Gly Met Gln Pro Gly Val Lys Ala Val Gln Val Val
            180                 185                 190
Leu Arg Ile Cys Tyr Ser Asp Thr Ser Cys Pro Gln Glu Asp Gln Tyr
        195                 200                 205
Pro Pro Asn Ile Ala Val Lys Val Asn His Ser Tyr Cys Ser Val Pro
    210                 215                 220
Gly Tyr Tyr Pro Ser Asn Lys Pro Gly Val Glu Pro Lys Arg Pro Cys
225                 230                 235                 240
Arg Pro Ile Asn Leu Thr His Leu Met Tyr Leu Ser Ser Ala Thr Asn

                245                 250                 255
Arg Ile Thr Val Thr Trp Gly Asn Tyr Gly Lys Ser Tyr Ser Val Ala
            260                 265                 270
Leu Tyr Leu Val Arg Gln Leu Thr Ser Ser Glu Leu Leu Gln Arg Leu
        275                 280                 285
Lys Thr Ile Gly Val Lys His Pro Glu Leu Cys Lys Ala Leu Val Lys
    290                 295                 300
Glu Lys Leu Arg Leu Asp Pro Asp Ser Glu Ile Ala Thr Thr Gly Val
305                 310                 315                 320
Arg Val Ser Leu Ile Cys Pro Leu Val Lys Met Arg Leu Ser Val Pro
                325                 330                 335
Cys Arg Ala Glu Thr Cys Ala His Leu Gln Cys Phe Asp Ala Val Phe
            340                 345                 350
Tyr Leu Gln Met Asn Glu Lys Lys Pro Thr Trp Met Cys Pro Val Cys
        355                 360                 365
Asp Lys Pro Ala Pro Tyr Asp Gln Leu Ile Ile Asp Gly Leu Leu Ser
    370                 375                 380
Lys Ile Leu Ser Glu Cys Glu Asp Ala Asp Glu Ile Glu Tyr Leu Val
385                 390                 395                 400
Asp Gly Ser Trp Cys Pro Ile Arg Ala Glu Lys Glu Arg Ser Cys Ser
                405                 410                 415
Pro Gln Gly Ala Ile Leu Val Leu Gly Pro Ser Asp Ala Asn Gly Leu
            420                 425                 430
Leu Pro Ala Pro Ser Val Asn Gly Ser Gly Ala Leu Gly Ser Thr Gly
        435                 440                 445
Gly Gly Gly Pro Val Gly Ser Met Glu Asn Gly Lys Pro Gly Ala Asp
    450                 455                 460
Val Val Asp Leu Thr Leu Asp Ser Ser Ser Ser Ser Glu Asp Glu Glu

465                 470                 475                 480

Glu Glu Glu Glu Glu Glu Glu Asp Glu Asp Glu Glu Gly Pro Arg Pro

                485                 490                 495

Lys Arg Arg Cys Pro Phe Gln Lys Gly Leu Val Pro Ala Cys

            500                 505                 510

<210> 13

<211> 1719

<212> DNA

<213> Homo sapiens

<220>

<221> CDS

<222> (1)..(1719)

<400> 13

atg gcg gat ttc gaa gag ttg agg aat atg gtt tct agt ttt agg gtt    48

Met Ala Asp Phe Glu Glu Leu Arg Asn Met Val Ser Ser Phe Arg Val

  1               5                  10                  15

tct gaa cta caa gta tta cta ggc ttt gct gga cgg aat aaa agt gga    96

Ser Glu Leu Gln Val Leu Leu Gly Phe Ala Gly Arg Asn Lys Ser Gly

                 20                  25                  30

cgc aag cat gac ctc ctg atg agg gcg ctg cat tta ttg aag agc ggc    144

Arg Lys His Asp Leu Leu Met Arg Ala Leu His Leu Leu Lys Ser Gly

         35                  40                  45

tgc agc cct gcg gtt cag att aaa atc cga gaa ttg tat aga cgc cga    192

Cys Ser Pro Ala Val Gln Ile Lys Ile Arg Glu Leu Tyr Arg Arg Arg

    50                  55                  60

tat cca cga act ctt gaa gga ctt tct gat tta tcc aca atc aaa tca 240

Tyr Pro Arg Thr Leu Glu Gly Leu Ser Asp Leu Ser Thr Ile Lys Ser

65                  70                  75                  80

tcg gtt ttc agt ttg gat ggt ggc tca tca cct gta gaa cct gac ttg 288

Ser Val Phe Ser Leu Asp Gly Gly Ser Ser Pro Val Glu Pro Asp Leu

                85                  90                  95

gcc gtg gct gga atc cac tcg ttg cct tcc act tca gtt aca cct cac 336

Ala Val Ala Gly Ile His Ser Leu Pro Ser Thr Ser Val Thr Pro His

            100                 105                 110

tca cca tcc tct cct gtt ggt tct gtg ctg ctt caa gat act aag ccc 384

Ser Pro Ser Ser Pro Val Gly Ser Val Leu Leu Gln Asp Thr Lys Pro

        115                 120                 125

aca ttt gag atg cag cag cca tct ccc cca att cct cct gtc cat cct 432

Thr Phe Glu Met Gln Gln Pro Ser Pro Pro Ile Pro Pro Val His Pro

    130                 135                 140

gat gtg cag tta aaa aat ctg ccc ttt tat gat gtc ctt gat gtt ctc 480

Asp Val Gln Leu Lys Asn Leu Pro Phe Tyr Asp Val Leu Asp Val Leu

145                 150                 155                 160

atc aag ccc acg agt tta gtt caa agc agt att cag cga ttt caa gag 528

Ile Lys Pro Thr Ser Leu Val Gln Ser Ser Ile Gln Arg Phe Gln Glu

                165                 170                 175

aag ttt ttt att ttt gct ttg aca cct caa caa gtt aga gag ata tgc 576

Lys Phe Phe Ile Phe Ala Leu Thr Pro Gln Gln Val Arg Glu Ile Cys

            180                 185                 190

ata tcc agg gat ttt ttg cca ggt ggt agg aga gat tat aca gtc caa 624

Ile Ser Arg Asp Phe Leu Pro Gly Gly Arg Arg Asp Tyr Thr Val Gln

        195                 200                 205

gtt cag ttg aga ctt tgc ctg gca gag aca agt tgc cct caa gaa gat    672
Val Gln Leu Arg Leu Cys Leu Ala Glu Thr Ser Cys Pro Gln Glu Asp
    210                 215                 220
aac tat cca aat agt cta tgt ata aaa gta aat ggg aag cta ttt cct    720
Asn Tyr Pro Asn Ser Leu Cys Ile Lys Val Asn Gly Lys Leu Phe Pro
225                 230                 235                 240
ttg cct ggc tat gca cca ccg cct aaa aat ggg att gaa cag aag cgc    768
Leu Pro Gly Tyr Ala Pro Pro Pro Lys Asn Gly Ile Glu Gln Lys Arg
                245                 250                 255
cct gga cgc ccc ttg aat att aca tct tta gtt agg tta tct tca gct    816
Pro Gly Arg Pro Leu Asn Ile Thr Ser Leu Val Arg Leu Ser Ser Ala
            260                 265                 270
gtg cca aac caa att tcc att tct tgg gca tca gaa att ggg aag aat    864
Val Pro Asn Gln Ile Ser Ile Ser Trp Ala Ser Glu Ile Gly Lys Asn
        275                 280                 285
tac tct atg tct gta tat ctt gta cgg cag ctt aca tca gcc atg tta    912
Tyr Ser Met Ser Val Tyr Leu Val Arg Gln Leu Thr Ser Ala Met Leu
    290                 295                 300
tta cag aga tta aaa atg aaa ggt att aga aac cct gat cat tcc aga    960
Leu Gln Arg Leu Lys Met Lys Gly Ile Arg Asn Pro Asp His Ser Arg
305                 310                 315                 320
gca cta att aaa gaa aaa ctt act gca gat cct gat agt gaa att gct    1008
Ala Leu Ile Lys Glu Lys Leu Thr Ala Asp Pro Asp Ser Glu Ile Ala
                325                 330                 335
aca act agc ctt cgg gta tcc ttg atg tgc cct tta gga aaa atg agg    1056
Thr Thr Ser Leu Arg Val Ser Leu Met Cys Pro Leu Gly Lys Met Arg
            340                 345                 350
ctg aca atc cca tgc cgt gca gtg act tgt aca cat ctg cag tgt ttt    1104

Leu Thr Ile Pro Cys Arg Ala Val Thr Cys Thr His Leu Gln Cys Phe
        355                 360                 365
gat gct gcc ctc tat cta caa atg aat gag aaa aag ccc acc tgg att 1152
Asp Ala Ala Leu Tyr Leu Gln Met Asn Glu Lys Lys Pro Thr Trp Ile
    370                 375                 380
tgt cct gtg tgt gac aaa aaa gct gcc tat gaa agt cta ata tta gat 1200
Cys Pro Val Cys Asp Lys Lys Ala Ala Tyr Glu Ser Leu Ile Leu Asp
385                 390                 395                 400
ggg ctt ttt atg gaa att ctc aat gac tgt tct gat gta gat gag atc 1248
Gly Leu Phe Met Glu Ile Leu Asn Asp Cys Ser Asp Val Asp Glu Ile
                405                 410                 415
aaa ttc caa gaa gat ggt tct tgg tgt cca atg aga ccg aag aaa gaa 1296
Lys Phe Gln Glu Asp Gly Ser Trp Cys Pro Met Arg Pro Lys Lys Glu
            420                 425                 430
gct atg aaa gta tcc agc caa ccg tgt aca aaa ata gaa agt tca agc 1344
Ala Met Lys Val Ser Ser Gln Pro Cys Thr Lys Ile Glu Ser Ser Ser
        435                 440                 445
gtc ctc agt aag cct tgt tca gtg act gta gcc agt gag gca agc aag 1392
Val Leu Ser Lys Pro Cys Ser Val Thr Val Ala Ser Glu Ala Ser Lys
    450                 455                 460
aag aaa gta gat gtt att gat ctt aca ata gaa agc tct tct gac gaa 1440
Lys Lys Val Asp Val Ile Asp Leu Thr Ile Glu Ser Ser Ser Asp Glu
465                 470                 475                 480
gag gaa gac cct cct gcc aaa agg aaa tgc atc ttt atg tca gaa aca 1488
Glu Glu Asp Pro Pro Ala Lys Arg Lys Cys Ile Phe Met Ser Glu Thr
                485                 490                 495
caa agc agc cca acc aaa ggg gtt ctc atg tat cag cca tct tct gta 1536
Gln Ser Ser Pro Thr Lys Gly Val Leu Met Tyr Gln Pro Ser Ser Val

500                 505                 510

agg gtg ccc agt gtg act tcg gtt gat cct gct gct att ccg cct tca     1584

Arg Val Pro Ser Val Thr Ser Val Asp Pro Ala Ala Ile Pro Pro Ser

        515                 520                 525

tta aca gac tac tca gta cca ttc cac cat acg cca ata tca agc atg     1632

Leu Thr Asp Tyr Ser Val Pro Phe His His Thr Pro Ile Ser Ser Met

    530                 535                 540

tca tca gat ttg cca gga gaa caa aga aga aat gat att aat aat gaa     1680

Ser Ser Asp Leu Pro Gly Glu Gln Arg Arg Asn Asp Ile Asn Asn Glu

545                 550                 555                 560

ctg aag ctt gga aca tct tct gat act gtg caa cag tga                 1719

Leu Lys Leu Gly Thr Ser Ser Asp Thr Val Gln Gln

                565                 570

<210> 14

<211> 572

<212> PRT

<213> Homo sapiens

<400> 14

Met Ala Asp Phe Glu Glu Leu Arg Asn Met Val Ser Ser Phe Arg Val

 1               5                  10                  15

Ser Glu Leu Gln Val Leu Leu Gly Phe Ala Gly Arg Asn Lys Ser Gly

            20                  25                  30

Arg Lys His Asp Leu Leu Met Arg Ala Leu His Leu Leu Lys Ser Gly

        35                  40                  45

Cys Ser Pro Ala Val Gln Ile Lys Ile Arg Glu Leu Tyr Arg Arg Arg

50                  55                  60

Tyr Pro Arg Thr Leu Glu Gly Leu Ser Asp Leu Ser Thr Ile Lys Ser

65                  70                  75                  80

Ser Val Phe Ser Leu Asp Gly Gly Ser Ser Pro Val Glu Pro Asp Leu

                85                  90                  95

Ala Val Ala Gly Ile His Ser Leu Pro Ser Thr Ser Val Thr Pro His

            100                 105                 110

Ser Pro Ser Ser Pro Val Gly Ser Val Leu Leu Gln Asp Thr Lys Pro

        115                 120                 125

Thr Phe Glu Met Gln Gln Pro Ser Pro Pro Ile Pro Pro Val His Pro

    130                 135                 140

Asp Val Gln Leu Lys Asn Leu Pro Phe Tyr Asp Val Leu Asp Val Leu

145                 150                 155                 160

Ile Lys Pro Thr Ser Leu Val Gln Ser Ser Ile Gln Arg Phe Gln Glu

                165                 170                 175

Lys Phe Phe Ile Phe Ala Leu Thr Pro Gln Gln Val Arg Glu Ile Cys

            180                 185                 190

Ile Ser Arg Asp Phe Leu Pro Gly Gly Arg Arg Asp Tyr Thr Val Gln

        195                 200                 205

Val Gln Leu Arg Leu Cys Leu Ala Glu Thr Ser Cys Pro Gln Glu Asp

    210                 215                 220

Asn Tyr Pro Asn Ser Leu Cys Ile Lys Val Asn Gly Lys Leu Phe Pro

225                 230                 235                 240

Leu Pro Gly Tyr Ala Pro Pro Pro Lys Asn Gly Ile Glu Gln Lys Arg

                245                 250                 255

Pro Gly Arg Pro Leu Asn Ile Thr Ser Leu Val Arg Leu Ser Ser Ala

            260                 265                 270

Val Pro Asn Gln Ile Ser Ile Ser Trp Ala Ser Glu Ile Gly Lys Asn

        275                 280                 285
Tyr Ser Met Ser Val Tyr Leu Val Arg Gln Leu Thr Ser Ala Met Leu
    290                 295                 300
Leu Gln Arg Leu Lys Met Lys Gly Ile Arg Asn Pro Asp His Ser Arg
305                 310                 315                 320
Ala Leu Ile Lys Glu Lys Leu Thr Ala Asp Pro Asp Ser Glu Ile Ala
                325                 330                 335
Thr Thr Ser Leu Arg Val Ser Leu Met Cys Pro Leu Gly Lys Met Arg
            340                 345                 350
Leu Thr Ile Pro Cys Arg Ala Val Thr Cys Thr His Leu Gln Cys Phe
        355                 360                 365
Asp Ala Ala Leu Tyr Leu Gln Met Asn Glu Lys Lys Pro Thr Trp Ile
    370                 375                 380
Cys Pro Val Cys Asp Lys Lys Ala Ala Tyr Glu Ser Leu Ile Leu Asp
385                 390                 395                 400
Gly Leu Phe Met Glu Ile Leu Asn Asp Cys Ser Asp Val Asp Glu Ile
                405                 410                 415
Lys Phe Gln Glu Asp Gly Ser Trp Cys Pro Met Arg Pro Lys Lys Glu
            420                 425                 430
Ala Met Lys Val Ser Ser Gln Pro Cys Thr Lys Ile Glu Ser Ser Ser
        435                 440                 445
Val Leu Ser Lys Pro Cys Ser Val Thr Val Ala Ser Glu Ala Ser Lys
    450                 455                 460
Lys Lys Val Asp Val Ile Asp Leu Thr Ile Glu Ser Ser Ser Asp Glu
465                 470                 475                 480
Glu Glu Asp Pro Pro Ala Lys Arg Lys Cys Ile Phe Met Ser Glu Thr
                485                 490                 495
Gln Ser Ser Pro Thr Lys Gly Val Leu Met Tyr Gln Pro Ser Ser Val

            500                 505                 510
Arg Val Pro Ser Val Thr Ser Val Asp Pro Ala Ala Ile Pro Pro Ser
        515                 520                 525
Leu Thr Asp Tyr Ser Val Pro Phe His His Thr Pro Ile Ser Ser Met
    530                 535                 540
Ser Ser Asp Leu Pro Gly Glu Gln Arg Arg Asn Asp Ile Asn Asn Glu
545                 550                 555                 560
Leu Lys Leu Gly Thr Ser Ser Asp Thr Val Gln Gln
                565                 570

<210> 15
<211> 1866
<212> DNA
<213> Homo sapiens

<220>
<221> CDS
<222> (1)..(1866)

<400> 15
atg gcg gat ttc gaa gag ttg agg aat atg gtt tct agt ttt agg gtt 48
Met Ala Asp Phe Glu Glu Leu Arg Asn Met Val Ser Ser Phe Arg Val
1               5                   10                  15
tct gaa cta caa gta tta cta ggc ttt gct gga cgg aat aaa agt gga 96
Ser Glu Leu Gln Val Leu Leu Gly Phe Ala Gly Arg Asn Lys Ser Gly
            20                  25                  30
cgc aag cat gac ctc ctg atg agg gcg ctg cat tta ttg aag agc ggc 144

Arg Lys His Asp Leu Leu Met Arg Ala Leu His Leu Leu Lys Ser Gly
        35                  40                  45
tgc agc cct gcg gtt cag att aaa atc cga gaa ttg tat aga cgc cga 192
Cys Ser Pro Ala Val Gln Ile Lys Ile Arg Glu Leu Tyr Arg Arg Arg
    50                  55                  60
tat cca cga act ctt gaa gga ctt tct gat tta tcc aca atc aaa tca 240
Tyr Pro Arg Thr Leu Glu Gly Leu Ser Asp Leu Ser Thr Ile Lys Ser
65                  70                  75                  80
tcg gtt ttc agt ttg gat ggt ggc tca tca cct gta gaa cct gac ttg 288
Ser Val Phe Ser Leu Asp Gly Gly Ser Ser Pro Val Glu Pro Asp Leu
                85                  90                  95
gcc gtg gct gga atc cac tcg ttg cct tcc act tca gtt aca cct cac 336
Ala Val Ala Gly Ile His Ser Leu Pro Ser Thr Ser Val Thr Pro His
            100                 105                 110
tca cca tcc tct cct gtt ggt tct gtg ctg ctt caa gat act aag ccc 384
Ser Pro Ser Ser Pro Val Gly Ser Val Leu Leu Gln Asp Thr Lys Pro
        115                 120                 125
aca ttt gag atg cag cag cca tct ccc cca att cct cct gtc cat cct 432
Thr Phe Glu Met Gln Gln Pro Ser Pro Pro Ile Pro Pro Val His Pro
    130                 135                 140
gat gtg cag tta aaa aat ctg ccc ttt tat gat gtc ctt gat gtt ctc 480
Asp Val Gln Leu Lys Asn Leu Pro Phe Tyr Asp Val Leu Asp Val Leu
145                 150                 155                 160
atc aag ccc acg agt tta gtt caa agc agt att cag cga ttt caa gag 528
Ile Lys Pro Thr Ser Leu Val Gln Ser Ser Ile Gln Arg Phe Gln Glu
                165                 170                 175
aag ttt ttt att ttt gct ttg aca cct caa caa gtt aga gag ata tgc 576
Lys Phe Phe Ile Phe Ala Leu Thr Pro Gln Gln Val Arg Glu Ile Cys

            180                 185                 190
ata tcc agg gat ttt ttg cca ggt ggt agg aga gat tat aca gtc caa 624
Ile Ser Arg Asp Phe Leu Pro Gly Gly Arg Arg Asp Tyr Thr Val Gln
        195                 200                 205
gtt cag ttg aga ctt tgc ctg gca gag aca agt tgc cct caa gaa gat 672
Val Gln Leu Arg Leu Cys Leu Ala Glu Thr Ser Cys Pro Gln Glu Asp
    210                 215                 220
aac tat cca aat agt cta tgt ata aaa gta aat ggg aag cta ttt cct 720
Asn Tyr Pro Asn Ser Leu Cys Ile Lys Val Asn Gly Lys Leu Phe Pro
225                 230                 235                 240
ttg cct ggc tat gca cca ccg cct aaa aat ggg att gaa cag aag cgc 768
Leu Pro Gly Tyr Ala Pro Pro Pro Lys Asn Gly Ile Glu Gln Lys Arg
                245                 250                 255
cct gga cgc ccc ttg aat att aca tct tta gtt agg tta tct tca gct 816
Pro Gly Arg Pro Leu Asn Ile Thr Ser Leu Val Arg Leu Ser Ser Ala
            260                 265                 270
gtg cca aac caa att tcc att tct tgg gca tca gaa att ggg aag aat 864
Val Pro Asn Gln Ile Ser Ile Ser Trp Ala Ser Glu Ile Gly Lys Asn
        275                 280                 285
tac tct atg tct gta tat ctt gta cgg cag ctt aca tca gcc atg tta 912
Tyr Ser Met Ser Val Tyr Leu Val Arg Gln Leu Thr Ser Ala Met Leu
    290                 295                 300
tta cag aga tta aaa atg aaa ggt att aga aac cct gat cat tcc aga 960
Leu Gln Arg Leu Lys Met Lys Gly Ile Arg Asn Pro Asp His Ser Arg
305                 310                 315                 320
gca cta att aaa gaa aaa ctt act gca gat cct gat agt gaa att gct 1008
Ala Leu Ile Lys Glu Lys Leu Thr Ala Asp Pro Asp Ser Glu Ile Ala
                325                 330                 335

aca act agc ctt cgg gta tcc ttg atg tgc cct tta gga aaa atg agg 1056
Thr Thr Ser Leu Arg Val Ser Leu Met Cys Pro Leu Gly Lys Met Arg
            340                 345                 350
ctg aca atc cca tgc cgt gca gtg act tgt aca cat ctg cag tgt ttt 1104
Leu Thr Ile Pro Cys Arg Ala Val Thr Cys Thr His Leu Gln Cys Phe
        355                 360                 365
gat gct gcc ctc tat cta caa atg aat gag aaa aag ccc acc tgg att 1152
Asp Ala Ala Leu Tyr Leu Gln Met Asn Glu Lys Lys Pro Thr Trp Ile
    370                 375                 380
tgt cct gtg tgt gac aaa aaa gct gcc tat gaa agt cta ata tta gat 1200
Cys Pro Val Cys Asp Lys Lys Ala Ala Tyr Glu Ser Leu Ile Leu Asp
385                 390                 395                 400
ggg ctt ttt atg gaa att ctc aat gac tgt tct gat gta gat gag atc 1248
Gly Leu Phe Met Glu Ile Leu Asn Asp Cys Ser Asp Val Asp Glu Ile
                405                 410                 415
aaa ttc caa gaa gat ggt tct tgg tgt cca atg aga ccg aag aaa gaa 1296
Lys Phe Gln Glu Asp Gly Ser Trp Cys Pro Met Arg Pro Lys Lys Glu
            420                 425                 430
gct atg aaa gta tcc agc caa ccg tgt aca aaa ata gaa agt tca agc 1344
Ala Met Lys Val Ser Ser Gln Pro Cys Thr Lys Ile Glu Ser Ser Ser
        435                 440                 445
gtc ctc agt aag cct tgt tca gtg act gta gcc agt gag gca agc aag 1392
Val Leu Ser Lys Pro Cys Ser Val Thr Val Ala Ser Glu Ala Ser Lys
    450                 455                 460
aag aaa gta gat gtt att gat ctt aca ata gaa agc tct tct gac gaa 1440
Lys Lys Val Asp Val Ile Asp Leu Thr Ile Glu Ser Ser Ser Asp Glu
465                 470                 475                 480
gag gaa gac cct cct gcc aaa agg aaa tgc atc ttt atg tca gaa aca 1488

Glu Glu Asp Pro Pro Ala Lys Arg Lys Cys Ile Phe Met Ser Glu Thr
                485                 490                 495
caa agc agc cca acc aaa ggg gtt ctc atg tat cag cca tct tct gta 1536
Gln Ser Ser Pro Thr Lys Gly Val Leu Met Tyr Gln Pro Ser Ser Val
            500                 505                 510
agg gtg ccc agt gtg act tcg gtt gat cct gct gct att ccg cct tca 1584
Arg Val Pro Ser Val Thr Ser Val Asp Pro Ala Ala Ile Pro Pro Ser
        515                 520                 525
tta aca gac tac tca gta cca ttc cac cat acg cca ata tca agc atg 1632
Leu Thr Asp Tyr Ser Val Pro Phe His His Thr Pro Ile Ser Ser Met
    530                 535                 540
tca tca gat ttg cca ggt ttg gat ttt ctt tcc ctt att cca gtt gat 1680
Ser Ser Asp Leu Pro Gly Leu Asp Phe Leu Ser Leu Ile Pro Val Asp
545                 550                 555                 560
ccc cag tac tgt cct cct atg ttt ttg gat agt ctc acc tca ccc tta 1728
Pro Gln Tyr Cys Pro Pro Met Phe Leu Asp Ser Leu Thr Ser Pro Leu
                565                 570                 575
aca gca agc agt acg tct gtc acc acc acc agc tcc cat gaa agc agt 1776
Thr Ala Ser Ser Thr Ser Val Thr Thr Thr Ser Ser His Glu Ser Ser
            580                 585                 590
act cat gtt agt tca tcc agc agc agg agt gag aca ggg gtc ata acc 1824
Thr His Val Ser Ser Ser Ser Ser Arg Ser Glu Thr Gly Val Ile Thr
        595                 600                 605
agc agt gga agt aac att cct gaa atc atc tca ttg gac taa 1866
Ser Ser Gly Ser Asn Ile Pro Glu Ile Ile Ser Leu Asp
    610                 615                 620

<210> 16

<211> 621

<212> PRT

<213> Homo sapiens

<400> 16

Met Ala Asp Phe Glu Glu Leu Arg Asn Met Val Ser Ser Phe Arg Val
1               5                   10                  15

Ser Glu Leu Gln Val Leu Leu Gly Phe Ala Gly Arg Asn Lys Ser Gly
            20                  25                  30

Arg Lys His Asp Leu Leu Met Arg Ala Leu His Leu Leu Lys Ser Gly
        35                  40                  45

Cys Ser Pro Ala Val Gln Ile Lys Ile Arg Glu Leu Tyr Arg Arg Arg
    50                  55                  60

Tyr Pro Arg Thr Leu Glu Gly Leu Ser Asp Leu Ser Thr Ile Lys Ser
65                  70                  75                  80

Ser Val Phe Ser Leu Asp Gly Gly Ser Ser Pro Val Glu Pro Asp Leu
                85                  90                  95

Ala Val Ala Gly Ile His Ser Leu Pro Ser Thr Ser Val Thr Pro His
            100                 105                 110

Ser Pro Ser Ser Pro Val Gly Ser Val Leu Leu Gln Asp Thr Lys Pro
        115                 120                 125

Thr Phe Glu Met Gln Gln Pro Ser Pro Pro Ile Pro Pro Val His Pro
    130                 135                 140

Asp Val Gln Leu Lys Asn Leu Pro Phe Tyr Asp Val Leu Asp Val Leu
145                 150                 155                 160

Ile Lys Pro Thr Ser Leu Val Gln Ser Ser Ile Gln Arg Phe Gln Glu
                165                 170                 175

Lys Phe Phe Ile Phe Ala Leu Thr Pro Gln Gln Val Arg Glu Ile Cys
            180                 185                 190
Ile Ser Arg Asp Phe Leu Pro Gly Gly Arg Arg Asp Tyr Thr Val Gln
        195                 200                 205
Val Gln Leu Arg Leu Cys Leu Ala Glu Thr Ser Cys Pro Gln Glu Asp
    210                 215                 220
Asn Tyr Pro Asn Ser Leu Cys Ile Lys Val Asn Gly Lys Leu Phe Pro
225                 230                 235                 240
Leu Pro Gly Tyr Ala Pro Pro Pro Lys Asn Gly Ile Glu Gln Lys Arg
                245                 250                 255
Pro Gly Arg Pro Leu Asn Ile Thr Ser Leu Val Arg Leu Ser Ser Ala
            260                 265                 270
Val Pro Asn Gln Ile Ser Ile Ser Trp Ala Ser Glu Ile Gly Lys Asn
        275                 280                 285
Tyr Ser Met Ser Val Tyr Leu Val Arg Gln Leu Thr Ser Ala Met Leu
    290                 295                 300
Leu Gln Arg Leu Lys Met Lys Gly Ile Arg Asn Pro Asp His Ser Arg
305                 310                 315                 320
Ala Leu Ile Lys Glu Lys Leu Thr Ala Asp Pro Asp Ser Glu Ile Ala
                325                 330                 335
Thr Thr Ser Leu Arg Val Ser Leu Met Cys Pro Leu Gly Lys Met Arg
            340                 345                 350
Leu Thr Ile Pro Cys Arg Ala Val Thr Cys Thr His Leu Gln Cys Phe
        355                 360                 365
Asp Ala Ala Leu Tyr Leu Gln Met Asn Glu Lys Lys Pro Thr Trp Ile
    370                 375                 380
Cys Pro Val Cys Asp Lys Lys Ala Ala Tyr Glu Ser Leu Ile Leu Asp
385                 390                 395                 400

Gly Leu Phe Met Glu Ile Leu Asn Asp Cys Ser Asp Val Asp Glu Ile
                405                 410                 415
Lys Phe Gln Glu Asp Gly Ser Trp Cys Pro Met Arg Pro Lys Lys Glu
            420                 425                 430
Ala Met Lys Val Ser Ser Gln Pro Cys Thr Lys Ile Glu Ser Ser Ser
        435                 440                 445
Val Leu Ser Lys Pro Cys Ser Val Thr Val Ala Ser Glu Ala Ser Lys
    450                 455                 460
Lys Lys Val Asp Val Ile Asp Leu Thr Ile Glu Ser Ser Ser Asp Glu
465                 470                 475                 480
Glu Glu Asp Pro Pro Ala Lys Arg Lys Cys Ile Phe Met Ser Glu Thr
                485                 490                 495
Gln Ser Ser Pro Thr Lys Gly Val Leu Met Tyr Gln Pro Ser Ser Val
            500                 505                 510
Arg Val Pro Ser Val Thr Ser Val Asp Pro Ala Ala Ile Pro Pro Ser
        515                 520                 525
Leu Thr Asp Tyr Ser Val Pro Phe His His Thr Pro Ile Ser Ser Met
    530                 535                 540
Ser Ser Asp Leu Pro Gly Leu Asp Phe Leu Ser Leu Ile Pro Val Asp
545                 550                 555                 560
Pro Gln Tyr Cys Pro Pro Met Phe Leu Asp Ser Leu Thr Ser Pro Leu
                565                 570                 575
Thr Ala Ser Ser Thr Ser Val Thr Thr Thr Ser Ser His Glu Ser Ser
            580                 585                 590
Thr His Val Ser Ser Ser Ser Ser Arg Ser Glu Thr Gly Val Ile Thr
        595                 600                 605
Ser Ser Gly Ser Asn Ile Pro Glu Ile Ile Ser Leu Asp
    610                 615                 620

<210> 17

<211> 2105

<212> DNA

<213> Homo sapiens

<220>

<221> CDS

<222> (94)..(1458)

<400> 17

gttgccatta ggggactcct gaggtcctat ctccaggctg cggtgactgc actttccctg 60

gagtggaagc tgctggaagg cggaccggcc gcc atg tcc acg ttc agg cag gag 114

Met Ser Thr Phe Arg Gln Glu

1               5

gac gtg gag gac cat tat gag atg ggg gag gag ctg ggc agc ggc cag 162

Asp Val Glu Asp His Tyr Glu Met Gly Glu Glu Leu Gly Ser Gly Gln

        10                  15                  20

ttt gcg atc gtg cgg aag tgc cgg cag aag ggc acg ggc aag gag tac 210

Phe Ala Ile Val Arg Lys Cys Arg Gln Lys Gly Thr Gly Lys Glu Tyr

    25                  30                  35

gca gcc aag ttc atc aag aag cgc cgc ctg tca tcc agc cgg cgt ggg 258

Ala Ala Lys Phe Ile Lys Lys Arg Arg Leu Ser Ser Ser Arg Arg Gly

40                  45                  50                  55

gtg agc cgg gag gag atc gag cgg gag gtg aac atc ctg cgg gag atc 306

Val Ser Arg Glu Glu Ile Glu Arg Glu Val Asn Ile Leu Arg Glu Ile

                60                  65                  70

cgg cac ccc aac atc atc acc ctg cac gac atc ttc gag aac aag acg    354
Arg His Pro Asn Ile Ile Thr Leu His Asp Ile Phe Glu Asn Lys Thr
            75                  80                  85
gac gtg gtc ctc atc ctg gag ctg gtc tct ggc ggg gag ctc ttt gac    402
Asp Val Val Leu Ile Leu Glu Leu Val Ser Gly Gly Glu Leu Phe Asp
        90                  95                  100
ttc ctg gcg gag aaa gag tcg ctg acg gag gac gag gcc acc cag ttc    450
Phe Leu Ala Glu Lys Glu Ser Leu Thr Glu Asp Glu Ala Thr Gln Phe
    105                 110                 115
ctc aag cag atc ctg gac ggc gtt cac tac ctg cac tct aag cgc atc    498
Leu Lys Gln Ile Leu Asp Gly Val His Tyr Leu His Ser Lys Arg Ile
120                 125                 130                 135
gca cac ttt gac ctg aag ccg gaa aac atc atg ctg ctg gac aag aac    546
Ala His Phe Asp Leu Lys Pro Glu Asn Ile Met Leu Leu Asp Lys Asn
                140                 145                 150
gtg ccc aac cca cga atc aag ctc atc gac ttc ggc atc gcg cac aag    594
Val Pro Asn Pro Arg Ile Lys Leu Ile Asp Phe Gly Ile Ala His Lys
            155                 160                 165
atc gag gcg ggg aac gag ttc aag aac atc ttc ggc acc ccg gag ttt    642
Ile Glu Ala Gly Asn Glu Phe Lys Asn Ile Phe Gly Thr Pro Glu Phe
        170                 175                 180
gtg gcc cca gag att gtg aac tat gag ccg ctg ggc ctg gag gcg gac    690
Val Ala Pro Glu Ile Val Asn Tyr Glu Pro Leu Gly Leu Glu Ala Asp
    185                 190                 195
atg tgg agc atc ggt gtc atc acc tat atc ctc ctg agc ggt gca tcc    738
Met Trp Ser Ile Gly Val Ile Thr Tyr Ile Leu Leu Ser Gly Ala Ser
200                 205                 210                 215
ccg ttc ctg ggc gag acc aag cag gag acg ctc acc aac atc tca gcc    786

Pro Phe Leu Gly Glu Thr Lys Gln Glu Thr Leu Thr Asn Ile Ser Ala
                220                 225                 230
gtg aac tac gac ttc gac gag gag tac ttc agc aac acc agc gag ctg     834
Val Asn Tyr Asp Phe Asp Glu Glu Tyr Phe Ser Asn Thr Ser Glu Leu
            235                 240                 245
gcc aag gac ttc att cgc cgg ctg ctc gtc aaa gat ccc aag cgg aga     882
Ala Lys Asp Phe Ile Arg Arg Leu Leu Val Lys Asp Pro Lys Arg Arg
        250                 255                 260
atg acc att gcc cag agc ctg gaa cat tcc tgg att aag gcg atc cgg     930
Met Thr Ile Ala Gln Ser Leu Glu His Ser Trp Ile Lys Ala Ile Arg
    265                 270                 275
cgg cgg aac gtg cgt ggt gag gac agc ggc cgc aag ccc gag cgg cgg     978
Arg Arg Asn Val Arg Gly Glu Asp Ser Gly Arg Lys Pro Glu Arg Arg
280                 285                 290                 295
cgc ctg aag acc acg cgt ctg aag gag tac acc atc aag tcg cac tcc     1026
Arg Leu Lys Thr Thr Arg Leu Lys Glu Tyr Thr Ile Lys Ser His Ser
                300                 305                 310
agc ttg ccg ccc aac aac agc tac gcc gac ttc gag cgc ttc tcc aag     1074
Ser Leu Pro Pro Asn Asn Ser Tyr Ala Asp Phe Glu Arg Phe Ser Lys
            315                 320                 325
gtg ctg gag gag gcg gcg gcc gcc gag gag ggc ctg cgc gag ctg cag     1122
Val Leu Glu Glu Ala Ala Ala Ala Glu Glu Gly Leu Arg Glu Leu Gln
        330                 335                 340
cgc agc cgg cgg ctc tgc cac gag gac gtg gag gcg ctg gcc gcc atc     1170
Arg Ser Arg Arg Leu Cys His Glu Asp Val Glu Ala Leu Ala Ala Ile
    345                 350                 355
tac gag gag aag gag gcc tgg tac cgc gag gag agc gac agc ctg ggc     1218
Tyr Glu Glu Lys Glu Ala Trp Tyr Arg Glu Glu Ser Asp Ser Leu Gly

360                 365                 370                 375

cag gac ctg cgg agg cta cgg cag gag ctg ctc aag acc gag gcg ctc    1266

Gln Asp Leu Arg Arg Leu Arg Gln Glu Leu Leu Lys Thr Glu Ala Leu

                380                 385                 390

aag cgg cag gcg cag gag gag gcc aag ggc gcg ctg ctg ggg acc agc    1314

Lys Arg Gln Ala Gln Glu Glu Ala Lys Gly Ala Leu Leu Gly Thr Ser

            395                 400                 405

ggc ctc aag cgc cgc ttc agc cgc ctg gag aac cgc tac gag gcg ctg    1362

Gly Leu Lys Arg Arg Phe Ser Arg Leu Glu Asn Arg Tyr Glu Ala Leu

        410                 415                 420

gcc aag caa gta gcc tcc gag atg cgc ttc gtg cag gac ctc gtg cgc    1410

Ala Lys Gln Val Ala Ser Glu Met Arg Phe Val Gln Asp Leu Val Arg

    425                 430                 435

gcc ctg gag cag gag aag ctg cag ggc gtg gag tgc ggg ctg cgc tag    1458

Ala Leu Glu Gln Glu Lys Leu Gln Gly Val Glu Cys Gly Leu Arg

440                 445                 450                 455

gcgcagtggg gtgggccagg ccccaggaca gccggagctc ggcctgcggt gggggcgctt 1518

cctgtggacg ctgcgcctcc catcgcccgg gtgcctgtcc ttgcccagcg ccaccaggct 1578

ggaggcggag tgggaggagc tggagccagg cccgtaagtt cgcaggcagg ggtgggtgtg 1638

ggacggggct gcttctctac acagcctcta cgctggcctt caccttcacc cctgcatcgt 1698

cggtgaccct gggaccctcc aggcagcgtg gcctgtggca ccgtgagggt tgggacccac 1758

cgaggcgcag aggcggcccg aatgcagccc tggttcaggc ccggaggagg gtttgcgggt 1818

agttgcacgg acaattcggc ggggtgctgc ctgttgctgc cattagccca ggaggaggtc 1878

gtgggacggg gagggtggga tggacggcgg acaggcagtc cccacgctgc tgggtggcgc 1938

cgggcttggt ggggtcttcc actgtgtgcc cttctcgccg aggccggtcc cccgggtgtg 1998

gggtgccctg ctgcggactc ctccgcgagc cccatcgtcg cgcctgtgga cgcctaggca 2058

agagcggccc tctgcagcca agagaaataa aatactggct tccagat                2105

<210> 18

<211> 454

<212> PRT

<213> Homo sapiens

<400> 18

Met Ser Thr Phe Arg Gln Glu Asp Val Glu Asp His Tyr Glu Met Gly
1               5                   10                  15

Glu Glu Leu Gly Ser Gly Gln Phe Ala Ile Val Arg Lys Cys Arg Gln
            20                  25                  30

Lys Gly Thr Gly Lys Glu Tyr Ala Ala Lys Phe Ile Lys Lys Arg Arg
        35                  40                  45

Leu Ser Ser Ser Arg Arg Gly Val Ser Arg Glu Glu Ile Glu Arg Glu
    50                  55                  60

Val Asn Ile Leu Arg Glu Ile Arg His Pro Asn Ile Ile Thr Leu His
65                  70                  75                  80

Asp Ile Phe Glu Asn Lys Thr Asp Val Val Leu Ile Leu Glu Leu Val
                85                  90                  95

Ser Gly Gly Glu Leu Phe Asp Phe Leu Ala Glu Lys Glu Ser Leu Thr
            100                 105                 110

Glu Asp Glu Ala Thr Gln Phe Leu Lys Gln Ile Leu Asp Gly Val His
        115                 120                 125

Tyr Leu His Ser Lys Arg Ile Ala His Phe Asp Leu Lys Pro Glu Asn
    130                 135                 140

Ile Met Leu Leu Asp Lys Asn Val Pro Asn Pro Arg Ile Lys Leu Ile
145                 150                 155                 160

Asp Phe Gly Ile Ala His Lys Ile Glu Ala Gly Asn Glu Phe Lys Asn

                165                 170                 175
Ile Phe Gly Thr Pro Glu Phe Val Ala Pro Glu Ile Val Asn Tyr Glu
            180                 185                 190
Pro Leu Gly Leu Glu Ala Asp Met Trp Ser Ile Gly Val Ile Thr Tyr
        195                 200                 205
Ile Leu Leu Ser Gly Ala Ser Pro Phe Leu Gly Glu Thr Lys Gln Glu
    210                 215                 220
Thr Leu Thr Asn Ile Ser Ala Val Asn Tyr Asp Phe Asp Glu Glu Tyr
225                 230                 235                 240
Phe Ser Asn Thr Ser Glu Leu Ala Lys Asp Phe Ile Arg Arg Leu Leu
                245                 250                 255
Val Lys Asp Pro Lys Arg Arg Met Thr Ile Ala Gln Ser Leu Glu His
            260                 265                 270
Ser Trp Ile Lys Ala Ile Arg Arg Arg Asn Val Arg Gly Glu Asp Ser
        275                 280                 285
Gly Arg Lys Pro Glu Arg Arg Arg Leu Lys Thr Thr Arg Leu Lys Glu
    290                 295                 300
Tyr Thr Ile Lys Ser His Ser Ser Leu Pro Pro Asn Asn Ser Tyr Ala
305                 310                 315                 320
Asp Phe Glu Arg Phe Ser Lys Val Leu Glu Glu Ala Ala Ala Ala Glu
                325                 330                 335
Glu Gly Leu Arg Glu Leu Gln Arg Ser Arg Arg Leu Cys His Glu Asp
            340                 345                 350
Val Glu Ala Leu Ala Ala Ile Tyr Glu Glu Lys Glu Ala Trp Tyr Arg
        355                 360                 365
Glu Glu Ser Asp Ser Leu Gly Gln Asp Leu Arg Arg Leu Arg Gln Glu
    370                 375                 380
Leu Leu Lys Thr Glu Ala Leu Lys Arg Gln Ala Gln Glu Glu Ala Lys

385                 390                 395                 400

Gly Ala Leu Leu Gly Thr Ser Gly Leu Lys Arg Arg Phe Ser Arg Leu

                405                 410                 415

Glu Asn Arg Tyr Glu Ala Leu Ala Lys Gln Val Ala Ser Glu Met Arg

            420                 425                 430

Phe Val Gln Asp Leu Val Arg Ala Leu Glu Gln Glu Lys Leu Gln Gly

        435                 440                 445

Val Glu Cys Gly Leu Arg

    450

<210> 19

<211> 66

<212> DNA

<213> Artificial Sequence

<220>

<223> Description of Artificial Sequence:synthetic DNA

<400> 19

tctagtcgac ggccagtgaa ttgtaatacg actcactata gggcgttttt tttttttttt 60

tttttt                                                            66

<210> 20

<211> 21

<212> DNA

<213> Artificial Sequence

<220>
<223> Description of Artificial Sequence:synthetic DNA

<400> 20
ctgcggtatg gcaactttca g                                                21

<210> 21
<211> 20
<212> DNA
<213> Artificial Sequence

<220>
<223> Description of Artificial Sequence:synthetic DNA

<400> 21
aggaatgcca agacatgcaa                                                  20

<210> 22
<211> 24
<212> DNA
<213> Artificial Sequence

<220>
<223> Description of Artificial Sequence:synthetic DNA

<400> 22

aactacggca agagctactc ggtg 24

<210> 23

<211> 24

<212> DNA

<213> Artificial Sequence

<220>

<223> Description of Artificial Sequence:synthetic DNA

<400> 23

gttcatctgc aggtagaaga cggc 24

## Description

### TECHNICAL FIELD

This invention relates to a method for detecting chronic myeloid leukemia; a method for evaluating a compound for its effectiveness in treating or preventing chronic myeloid leukemia; a therapeutic or prophylactic agent for chronic myeloid leukemia containing the compound obtained by the evaluation method; a therapeutic or prophylactic agent for chronic myeloid leukemia containing such compound; and a method for determining the risk of suffering from chronic myeloid leukemia.

### BACKGROUND ART

Chronic myeloid leukemia (CML) is a malignant disease caused by abnormal clonal growth of pluripotent hematopoietic stem cells [Kantarjian, H.M. et al.: Blood 82: 691(1993)]. Onset of this disease is believed to be caused by BCR-ABL fusion protein which is produced as a result of reciprocal translocation between chromosomes 9 and 22, and this BCR-ABL fusion protein is believed to act as an active tyrosine kinase to induce uncontrolled cell growth [Era, T. et al.: Proc. Natl. Acad. Sci. USA 97: 1737(2000)]. This uncontrolled cell growth results not only in the increase of undifferentiated blood cells but also in the differentiated blood cells from malignant clone, and as a consequence, the peripheral blood exhibits leukocytosis, thrombocytosis, and like.

The phase when the malignant clone has not yet lost its differentiation ability is called chronic phase (chronic phase: CP), and in this phase, the patients suffer from relatively mild clinical conditions. In most patients in their chronic phase, blood cell count of the peripheral blood can be controlled by administering interferon (IFN) α or hydroxyurea (HU) [Silver, R.T. et al.: Blood 94: 1517(1999)]. However, malignant blasts of many chronic myeloid leukemia patients lose their differentiation potency after several years, and as a result, the patients exhibits "acute leukemia" like conditions in which only the undifferentiated CML blasts propagate with the decrease in the mature blood cells. This phase is called blast crisis (blast crisis: BC) phase, and some patients are observed to experience accelerated phase (accelerated phase: AP) in the transition from the chronic phase to the blast crisis phase.

Once the patient is in the blast crisis phase, 50% survival period of the patient is generally as short as about 3 months, and the prognosis is extremely severe. Compared to the cells generally found in acute myeloid leukemia, CML blasts of the patient in the blast crisis phase are resistant to chemotherapy, and the prognosis is difficult to improve even by bone marrow transplantation. In order to improve the prognosis of the patients, it is therefore quite important to clarify the mechanism of the transition from the chronic phase to the accelerated phase or the blast crisis phase at the molecular level to thereby stop such transition. In contrast to the onset of the chronic myeloid leukemia defined by the production of the BCR-ABL fused gene product, the molecular mechanism that results in the advance into the accelerated phase or the blast crisis phase is little clarified. While activation of RAS oncogene product associated with point mutation [LeMaistre, A. et al.: Blood 73: 889(1989); Cogswell, P.C. et al.: Blood 74: 2629(1989)], and inactivation of p53 tumor suppressor gene product [Guran, S. et al.: Haematologia 29: 181(1998); Peller, S. et al.: Gene Chromosome Canc. 21: 2(1998)] have been reported to be associated with the advance of the disease phase, frequency of such events in the patient's specimen is not very high, and these events are unlikely to be the general mechanisms that are responsible for the advance of the chronic myeloid leukemia phase. While abnormality of chromosome 17 where p53 gene is located is often found in the late stage of chronic myeloid leukemia, Fioretos et al. describes that abnormality in the amino acid sequence of the p53 protein is rather rare in these cases, and Fioretos et al. postulates the presence of an unidentified tumor suppressor gene on the same chromosome whose gene abnormality plays an important role in the advance of the chronic myeloid leukemia phase [Fioretos, T. et al.: Blood 94: 225(1999)].

DNA microarray (also referred to as a DNA chip) is a recently developed system for analyzing gene expression, and this system has enabled macroscopic observation of gene expression of several thousands to tens of thousands of genes by repeating the experiment for several times. However, no report is so far known that has analyzed gene expression profile of the chronic myeloid leukemia patients by such system.

### DISCLOSURE OF THE INVENTION

An object of this invention is to provide a method for detecting chronic myeloid leukemia; a method for detecting risk factor for chronic myeloid leukemia; a method for evaluating a compound for its effectiveness in treating or preventing chronic myeloid leukemia; a compound having therapeutic or prophylactic effects obtained by such evaluation method; and a therapeutic or prophylactic agent for chronic myeloid leukemia containing such compound as its effective component.

The inventors of the present invention have made an extensive investigation to solve the problems as described above, and succeeded in identifying the genes whose expression level alters in phase-specific manner in the AC 133 protein-positive hematopoietic stem cell of the patients suffering from chronic myeloid leukemia. The present invention has been completed on the bases of such identification.

This invention provides a method for detecting chronic myeloid leukemia comprising the step of measuring expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3 gene in a test tissue or a test cell; or determining expression profile of a gene group containing at least one of said genes to thereby detect chronic myeloid leukemia. IFIT-2 gene is the gene for the interferon-induced protein represented by the nucleotide sequence shown in SEQ ID NO: 1 [Levy, Det al.: Proc. Natl. Acad. Sci. USA 83: 8929(1986)], or its allelic variant; LAGS-1 gene is the gene represented by the nucleotide sequence shown in SEQ ID NO: 3 [Lethe, B et al.: Int. J. Cancer 76: 903(1998)] or its allelic variant; BAGE gene is the gene represented by the nucleotide sequence shown in SEQ ID NO: 5 [Boel, P et al.: Immunity 2: 167(1995)] or its allelic variant; DDB1 gene is the gene for a DNA damage-binding protein represented by the nucleotide sequence shown in SEQ ID NO: 7 [Dualan, R et al.: Genomics 29: 62(1995)] or its allelic variant; ETS2 gene is the gene represented by the nucleotide sequence shown in SEQ ID NO: 9 [Watson, D.K. et al.: Proc. Natl. Acad. Sci. USA 85: 7862(1988)] or its allelic variant; PIASy gene is the gene represented by the nucleotide sequence shown in SEQ ID NO: 11 [Liu, B et al.: Proc. Natl. Acad. Sci. USA 95: 10626(1998)] or its allelic variant; PIASx-α gene is the gene represented by the nucleotide sequence shown in SEQ ID NO: 13 [Liu, B et al.: Proc. Natl. Acad. Sci. USA 95: 10626(1998)] or its allelic variant; PIASx-β gene is the gene represented by the nucleotide sequence shown in SEQ ID NO: 15 [Liu, B et al.: Proc. Natl. Acad. Sci. USA 95: 10626(1998)] or its allelic variant; and DAPK3 gene is the gene represented by the nucleotide sequence shown in SEQ ID NO: 17 [Kawai, T. et al.: Mol. Cell. Biol. 18: 1642(1998)] or its allelic variant. The genes as described above are summarized in Table 1, below.

**Table 1**

| Name of the gene | Accession No. (GenBank) | SEQ ID NO: (Nucleotide sequence) | SEQ ID NO: (Amino acid sequence) |
|---|---|---|---|
| IFIT-2 | M14660/X07557 | SEQ ID NO: 1 | SEQ ID NO: 2 |
| LAGE-1 | AJ223041 | SEQ ID NO: 3 | SEQ ID NO: 4 |
| BAGE | U19180 | SEQ ID NO: 5 | SEQ ID NO: 6 |
| DDB1 | U18299 | SEQ ID NO: 7 | SEQ ID NO: 8 |
| ETS-2 | J04102 | SEQ ID NO: 9 | SEQ ID NO: 10 |
| PIASy | AF077952 | SEQ ID NO: 11 | SEQ ID NO: 12 |
| PIASx-α | AF077953 | SEQ ID NO: 13 | SEQ ID NO: 14 |
| PIASx-β | AF077954 | SEQ ID NO: 15 | SEQ ID NO: 16 |
| DAPK3 | AB007144/NM001348 | SEQ ID NO: 17 | SEQ ID NO: 18 |

The test cell used may be a bone marrow cell or a blood cell, and in particular, an undifferentiated stem cell which propagates in the patient suffering from chronic myeloid leukemia, namely, a pluripotent hematopoietic stem cell (in particular, AC133 protein-positive hematopoietic stem cell). The measurement of the expression level of the gene or the determination of the expression profile of the gene group may be conducted at the level of either mRNA or protein. The measurement of the expression level of the gene or the determination of the expression profile of the gene group at the mRNA level may be conducted, for example, by gene amplification or by using a DNA microarray. The measurement of the expression level of the gene or the determination of the expression profile of the gene group at the protein level may be conducted, for example, by means of immunological method or by using a protein chip.

This invention also provides a method for determining phase of the chronic myeloid leukemia wherein the phase of the chronic myeloid leukemia is determined by using the detection method of the chronic myeloid leukemia according to any one of the detection methods as described above. The phase may be selected from chronic phase, accelerated phase, and blastic phase.

This invention also provides a method for evaluating a compound for its effectiveness in treating or preventing chronic myeloid leukemia, comprising the steps of:
(a) administering the compound to be evaluated to a test animal or a test cell, or bringing the compound in contact with a test animal or a test cell; and
(b) confirming whether the compound regulates expression level of a gene selected from the group consisting of IFIT-2 gene, LAGS-1 gene, BAGE gene, DDB1 gene, ETS2 gene, PIASy gene, PIASx-α gene, PIASx-β gene, and DAPK3 gene, or a gene which is functionally equivalent to such gene in test animal or the test cell.

This invention also provides a method for evaluating a compound for its effectiveness in treating or preventing chronic myeloid leukemia, comprising the steps of:
(a) bringing the compound to be evaluated in contact with a test animal or a test cell containing a fusion gene of an expression-regulatory region of a gene selected from the group consisting of IFIT-2 gene, LAGE-1 gene, BAGE gene, DDB1 gene, ETS2 gene, PIASy gene, PIASx-α gene, PIASx-β gene, and DAPK3 gene; and a reporter gene; and
(b) measuring the level of the reporter gene expressed in the test animal or the test cell to evaluate the compound.

This invention also provides a method for evaluating a compound for its effectiveness in treating or preventing chronic myeloid leukemia, comprising the steps of:
(a) bringing the compound to be evaluated in contact with a protein encoded by a gene selected from the group consisting of IFIT-2 gene, LAGE-1 gene, BAGE gene, DDB 1 gene, ETS2 gene, PIASy gene, PIASx-α gene, PIASx-β gene, and DAPK3 gene; and
(b) measuring the activity of the protein to evaluate the compound.

This invention also provides method for evaluating a compound for its effectiveness in treating or preventing chronic myeloid leukemia, comprising the steps of:
(a) bringing the compound to be evaluated in contact with a STAT protein whose activity is inhibited by a protein coded by PIASy gene or PIASx-β gene; and
(b) measuring the activity of the STAT protein to evaluate the compound.

This invention also provides a therapeutic or prophylactic agent for chronic myeloid leukemia containing the compound obtained by the evaluation method of any one of the evaluation method as described above as its effective component.

This invention also provides a reagent for research purpose, or an agent for detection, treatment, or prevention of chronic myeloid leukemia comprising an antibody directed against a protein encoded by a gene selected from the group consisting of IFIT-2 gene, LAGE-1 gene, BAGE gene, DDB1 gene, ETS2 gene, PIASy gene, PIASx-α gene, PIASx-β gene, and DAPK3 gene, as an active ingredient.

This invention also provides a method for determining the risk of being susceptible for chronic myeloid leukemia comprising the step of detecting genetic polymorphism which causes abnormality in expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3.

This invention also provides a method for determining the risk of being susceptible for chronic myeloid leukemia comprising the step of detecting genetic polymorphism which causes abnormality in biological activity of a protein encoded by a gene selected from the group consisting of IFIT-2 gene, LAGE-1 gene, BAGE gene, DDB 1 gene, ETS2 gene, PIASy gene, PIASx-α gene, PIASx-β gene, and DAPK3 gene.

This invention also provides a therapeutic or prophylactic agent for chronic myeloid leukemia comprising an antisense nucleic acid which is capable of specifically hybridizing with the mRNA of a gene selected from the group consisting of IFIT-2 gene, LAGE-1 gene, BAGE gene, DDB1 gene, and ETS2 gene.

This invention also provides a therapeutic or prophylactic agent for chronic myeloid leukemia comprising a ribozyme capable of specifically cleaving the mRNA of a gene selected from the group consisting of IFIT-2 gene, LAGE-1 gene, BAGE gene, DDB1 gene, and ETS2 gene.

Next, the present invention is described in further detail.

This invention relates to a unique method for detecting chronic myeloid leukemia which is quite different from conventional methods used for detecting chronic myeloid leukemia, wherein expression level of a particular gene or expression profile of a particular gene group in the test tissue or the test cell is used as an index for the detection of the chronic myeloid leukemia. The term "expression level" , as used herein, refers to an absolute or relative amount of the transcription product of the particular gene (hereinafter also referred to as the "mRNA level") or an absolute, or relative amount of the translation product of the particular gene (hereinafter also referred to as the "protein level"). The term "expression profile", as used herein, refers to a patternized profile of expression levels of a plurality of genes including the above-mentioned particular gene.

### 1. Detection method of chronic myeloid leukemia according to the present invention

The present invention has revealed that IFIT-2 gene, LAGE-1 gene, BAGE gene, DDB1 gene, ETS2 gene, PIASy gene, PIASx-α gene, PIASx-β gene, and DAPK3 gene exhibit unique alteration of expression level in the AC133-positive hematopoietic stem cell of patients suffering from chronic myeloid leukemia. Accordingly, these genes are useful as a marker gene for chronic myeloid leukemia, and chronic myeloid leukemia can be detected by measuring the expression level of such gene or by determining the expression profile of the gene group including such gene in the tissue from the donor (used interchangeably herein with "test tissue") or the cell from the donor (used interchangeably herein with "test cell"). The test cell used may be a bone marrow cells or a blood cell, and preferably a pluripotent hematopoietic stem cell, and most preferably an AC133-positive hematopoietic stem cell, an Flt3/Flk2 (GenBank XM007174)-positive hematopoietic stem cell, or a KDR/Flk (GenBank NM002253)-positive hematopoietic stem cell. While the test cell used in the Examples of the present invention is the AC133-positive hematopoietic stem cell from bone marrow, it is to be noted that the AC133-positive hematopoietic stem cell is also found in peripheral blood [Blood 95: 3106-3112 (2000)], and a blood cell can also be used in the detection method of the present invention.

Specifically, if the expression level of the gene as described above in the test tissue or the test cell is increased or decreased in comparison with the expression level in the chronic myeloid leukemia revealed by the present invention, the donor can be evaluated as suffering from the chronic myeloid leukemia. To be more specific, the genes as described above exhibit alteration of the expression level as described below in each phase of the chronic myeloid leukemia.

More specifically, the expression level of IFIT-2 gene increases in the patient of chronic phase chronic myeloid leukemia to the level of about 2 to 500 times higher than that of the normal donor while the expression level reduces again in the accelerated phase and the blast crisis phase. In the case of LAGE-1 gene and BAGE gene, the expression level in the normal donor is either zero or at a very low level if expressed, and the expression is found only in the chronic phase and the expression level again reduces in the accelerated phase and the blast crisis phase. Therefore, if the expression level of such gene in the hematopoietic stem cell of a donor is found to have increased to the level equivalent to the level as described above, the donor can be determined to be a patient of chronic myeloid leukemia and the phase of the disease can be evaluated to be the chronic phase.

In the hematopoietic stem cells of the patient suffering from chronic myeloid leukemia of the accelerated phase or the blast crisis phase, the expression level of DDB gene can increase to the level of about 20 to 50 times higher than that of the normal donor or the patient of chronic phase chronic myeloid leukemia, and/or the expression level of ETS2 gene can increase to the level of about 2 to 10 times higher than that of the normal donor or the patient of chronic phase chronic myeloid leukemia. Therefore, if the expression level of such gene in the hematopoietic stem cell of a donor is found to have increased to the level equivalent to the level as described above, the donor can be determined to be a patient of chronic myeloid leukemia and the phase of the disease can be evaluated to be the accelerated phase or the blast crisis phase.

In the hematopoietic stem cells of the patient suffering from chronic myeloid leukemia of the accelerated phase or the blast crisis phase, the expression level of PIASy gene, PIASx-α and β genes, and DAPK3 gene can uniquely decrease to the level of about 1/2 to 1/50. respectively, compared to the normal donor or the patient of chronic phase chronic myeloid leukemia. Therefore, if the expression level of such gene in the hematopoietic stem cell of a donor is found to have decreased to the level equivalent to the level as described above, the donor can be determined to be a patient of chronic myeloid leukemia and the phase of the disease can be evaluated to be the accelerated phase or the blast crisis phase.

The mRNA expression level for the gene can be measured by gene amplification (for example, by PCR or by real time RT-PCR), by using a DNA microarray, or by dot blot hybridization, slot blot hybridization, northern hybridization, or the like. The protein expression level for the gene can be measured by using a protein chip, by an immunological process (for example, by ELISA), or the like.

In addition, whether the donor suffers from chronic myeloid leukemia can be determined more accurately by specifying expression profile of a gene group including any one of the chronic myeloid leukemia marker genes for the biological sample from the donor, and comparing the resulting expression profile with the expression profile of the case which has been preliminarily identified as the expression profile of the chronic myeloid leukemia. In other words, by this means, even the unchanged expression level shown for a gene can also be monitored and utilized as a control in order to improve the accuracy and reliability of the measurement result. The expression profile is preferably determined by using a DNA microarray in view of convenience, sensitivity, reproducibility, and the like.

Furthermore, a DNA containing at least a part of the IFIT-2 gene, LAGS-1 gene, BAGE gene, DDB1 gene, ETS2 gene, PIASy gene, PIASx-α gene, PIASx-β gene, or DAPK3 gene can be used as a primer or a probe to analyze the gene or the mRNA of the gene. The term "at least a part", as used herein, means that the oligonucleotide which is used for the primer or the probe comprises a nucleotide sequence containing at least 8 nucleotides corresponding to the DNA sequence of the present invention, and preferably a nucleotide sequence of at least 10 nucleotides, and more preferably a polynucleotide containing at least about 15 to 25 nucleotides having the corresponding sequence.

### 2. Evaluation of the compound which may be effective in treating or preventing chronic myeloid leukemia

A substance, which is capable of normalizing the expression level or the activity level of the chronic myeloid leukemia-marker gene product thereby to enable differentiation of the leukemia cell into a normal cell or suppression of the abnormal growth of the leukemia cell, is expected to be found among some of the compounds which can regulate the expression level of a chronic myeloid leukemia marker gene, the activity of the chronic myeloid leukemia-marker gene product, or the STAT protein activity inhibited by the chronic myeloid leukemia-marker gene. On the bases of such expectation, compounds which may be effective in treating or preventing chronic myeloid leukemia may be determined by the procedure as described below. The term "evaluation", as used herein, is to a wide concept including both the screening and the validation of a compound.

### (1) Evaluation using expression level of chronic myeloid leukemia marker gene for the index

A test compound can be evaluated for its effectiveness in the treatment or prevention of the chronic myeloid leukemia by administering the test compound to or by bringing the test compound in contact with a test animal (for example, mouse, rat, or rabbit) or a test cell (for example, a human cell line of chronic myeloid leukemia, human blood cell, or AC133-positive hematopoietic stem cell), and confirming whether the test compound regulates the expression level of IFIT-2 gene, LAGE-1 gene, BAGE gene, DDB1 gene, ETS2 gene, PIASy gene, PIASx-α gene, PIASx-β gene, DAPK3 gene, or a gene which is functionally equivalent to these genes (i.e. whether the test compound brings the expression level of such a gene to the normal level) in the test animal or the test cell. The test animal or the test cell used may also be an animal or a cell having artificially introduced therein a fusion gene of the region regulating the expression of the IFIT-2 gene, LAGE-1 gene, BAGE gene, DDB1 gene, ETS2 gene, PIASy gene, PIASx-α gene, PIASx-β gene, or DAPK3 gene with a reporter gene . The reporter gene used is not limited to any specific gene, and exemplary reporter genes include β-galactosidase gene, luciferase gene, green fluorescence protein gene, and the like.

### (2) Evaluation using activity-regulating ability of the chronic myeloid leukemia marker gene for the index

A test compound can be evaluated for its effectiveness in the treatment or prevention of the chronic myeloid leukemia by bringing the test compound in contact with a protein coded by IFIT-2 gene, LAGE-1 gene, BAGE gene, DDB1 gene, ETS2 gene, PIASy gene, PIASx-α gene, PIASx-β gene, or DAPK3 gene, and confirming whether the test compound regulates the activity of the protein.

### (3) Evaluation using the ability of regulating the activity of STAT protein for the index

A test compound can be evaluated for its effectiveness on the treatment or prevention of the chronic myeloid leukemia by bringing the test compound in contact with a STAT protein whose activity is inhibited by the protein coded by PIASy gene, PIASx=α gene, or PIASx=β gene, and confirming that the test compound regulates the activity of the STAT protein.

### 3. Applications of the antibody against the product of a chronic myeloid leukemia marker gene

An antibody against the product of the chronic myeloid leukemia-marker gene can be produced by the procedure as described below. The term "antibody", as used herein, refers to the entire molecule of the antibody which binds to the product of a chronic myeloid leukemia-marker gene which is the antigen, as well as its fragment (for example, Fab or F(ab')₂ fragment). The antibody against the product of the chronic myeloid leukemia-marker gene can be produced by any of the techniques known in the art. For example, a monoclonal antibody can be isolated from the culture supernatant of the hybridoma or from the ascites of the mouse administered with the hybridoma, after preparing the cell fusion of a commercially available mouse myeloma cell with the splenocyte from the mouse immunized against the chronic myeloid leukemia-marker gene product. The monoclonal antibody can also be produced from an in vitro system using phage display method. The product of the chronic myeloid leukemia-marker gene used for the antigen does not necessarily have the entire amino acid structure, and may be a peptide having a partial amino acid structure, its variant, derivative, or fusion peptide with other peptides, and the product of the marker gene may be the one produced by any one of genetic engineering technique, biological process, and chemical synthesis. The antibody can be used as a research-purpose reagent used in identifying or quantitating the product of the chronic myeloid leukemia-marker gene in a human biological sample, or as an effective component in a diagnostic, therapeutic, or prophylactic agent for chronic myeloid leukemia. The antibody used in the present invention can also be used as a fragment such as Fab, F(ab')₂, or its derivative, or as a chimeric antibody or a humanized antibody, all of which are included within the present invention. The product of the chronic myeloid leukemia-marker gene can be immunologically measured by any of the methods known in the art including fluorescein antibody technique, passive hemagglutination, and enzyme antibody technique.

### 4. Application of the antisense nucleic acids, which can hybridize with the mRNAs of chronic myeloid leukemia marker gene, and the ribozymes capable of cleaving such mRNAs

Of the chronic myeloid leukemia marker genes revealed in this invention, IFIT-2 gene, LAGE-1 gene, BAGE gene, DDB 1 gene, and ETS2 gene are found to exhibit increased mRNA expression levels in the AC133-positive hematopoietic stem cells derived from chronic myeloid leukemia patients. In particular, the expression levels for the DDB1 gene and the ETS2 gene are found to increase in the transition from the accelerated phase to the blast crisis phase in proportion to the malignancy of the chronic myeloid leukemia. Therefore, an antisense nucleic acid (for example, DNA or RNA) which suppresses expression of these genes by specifically hybridizing with the mRNA of these genes, a gene construct expressing such antisense nucleic acid, a ribozyme which specifically cleaves such mRNA, and a gene construct expressing such ribozyme are candidates for the therapeutic agent of the chronic myeloid leukemia. The antisense nucleic acid, the ribozyme, and the gene constructs expressing such antisense nucleic acid or ribozyme can be produced by a method known in the art by using the nucleotide sequence of the IFIT-2 gene, LAGE-1 gene, BAGE gene, DDB1 gene, or ETS2 gene.

### 5. Therapeutic or prophylactic agent for chronic myeloid leukemia

Chronic myeloid leukemia marker genes are genes whose expression level markedly alters in the blood cell of the patient suffering from chronic myeloid leukemia. Therefore, a substance capable of regulating the expression level of the chronic myeloid leukemia marker gene so as to get it back to its normal level in the blood cell is useful for treating or preventing the chronic myeloid leukemia. Exemplary substances which regulate the expression level of the chronic myeloid leukemia marker gene in the blood cell back to the normal level include the compound evaluated in the above 2, the antibody obtained in the above 3, and the antisense nucleic acid, the ribozyme, and the gene constructs expressing these obtained in the above 4.

When such substance is used as a therapeutic or prophylactic agent for chronic myeloid leukemia, the substance can be formulated into an appropriate dosage form by mixing the substance with a carrier generally used in the art depending on the administration route. For example, in the case of oral administration, the substance may be formulated into a tablet, capsule, granule, powder, liquid, or other dosage form, and a solid formulation for oral administration may be prepared by using an excipient, a binder, or a lubricant, or in further combination with a colorant or a disintegrant. Exemplary excipients include lactose, starch, talc, magnesium stearate, crystalline cellulose, methyl cellulose, carboxymethylcellulose, glycerin, sodium alginate, and gum arabic. Exemplary binders include polyvinyl alcohol, polyvinylether, ethyl cellulose, gum arabic, shellac, and saccharose. Exemplary lubricants include magnesium stearate and talc. The colorant, disintegrant, and the like used may be those known in the art. The tablet may be coated by the method well known in the art. The liquid formulation may be an aqueous or oleaginous suspension, a solution, a syrup, a elixir, or the like which is prepared by the method generally used in the art. When an injection is prepared, the substance as described above may be mixed with a pH adjusting agent, a buffer, a stabilizer, an isotonic agent, a local anesthetic, or the like, and a subcutaneous, intramuscular, or intravenous injection may be produced by a conventional method. Exemplary bases used in producing a suppository include cacao butter, polyethylene glycol, and other oil base.

The dose of the resulting preparation can not be determined universally since the dose differs by the conditions, weight, age, and the like of the patient. However, the compound of the present invention is generally administered at a dose in the range of about 0.1 to 1000 mg, which is administered in 1 to 4 times a day. If desired, the administration may be accomplished at an interval of 1 to several days, or at an even longer interval.

### 6. Determination of susceptibility for chronic myeloid leukemia by utilizing genetic polymorphism

Liability to suffer from chronic myeloid leukemia (susceptibility for chronic myeloid leukemia) can be determined by detecting the genetic polymorphism which results in the abnormal expression of the marker gene for chronic myeloid revealed by the present invention, or by the polymorphism which results in the abnormal activity of the product of such gene. Exemplary genetic polymorphisms include minisatellite DNA, microsatellite DNA, and SNP (single nucleotide polymorphism), and such genetic polymorphism may be identified as described below. Namely, genetic polymorphism may be identified by sequencing the region of the chronic myeloid leukemia marker gene revealed by the present invention and the region which may be associated with the increase or decrease of the expression level of such gene in both the group of normal donors and the group of chronic rheumatism patients to thereby determine the polymorphic site, and then calculating the allele frequency of the polymorphic site thus determined and finding the allele whose frequency is significantly higher in the patient group to thereby identify the polymorphism correlated to the chronic myeloid leukemia.

The genetic polymorphism thus determined is preferably detected, for example, by determining the nucleotide sequence of the polymorphic site of the genomic DNA from the donor; by using a method which utilizes the difference in physicochemical nature of the DNA sample or the difference of the restriction site which depends on the changed nucleotide sequence for the polymorphic site (for example, by the method utilizing the difference in the mobility of the DNA sample including the polymorphic site in gel electrophoresis, capillary electrophoresis, or other electrophoretic means); by using a detection probe which is appropriate for detecting the particular polymorphic site; or by a method using mass spectroscopy. The method used is not limited to those described above as long as the method is capable of detecting the genetic polymorphism as described above, and any method known in the art can be employed for the detection. In any of the detection method as described above, it is to be noted that the DNA sample to be assayed is a sample of human origin, and the type of the sample is not limited as long as the sample contains the DNA to be assayed. Exemplary samples are body fluids such as blood, bone marrow, semen, ascites, and urine; cells from the tissue such as liver; hairs such as those on the head. Genomic DNA can be extracted, purified, and prepared from these samples by the method generally used in the art.

In the method for detecting chronic myeloid leukemia risk factor according to the present invention, the donor is determined to have a high risk of suffering from chronic myeloid leukemia when the polymorphism detected for the donor is the one whose frequency is significantly high in a chronic myeloid leukemia patient, and the donor is determined to have a low risk of suffering from chronic myeloid leukemia when the polymorphism detected is not such type.

### BRIEF DESCRIPTION OF DRAWINGS

FIG. 1 is a schematic view showing pMX-tetOFF/PIASy-F constructed in Example 5.
FIG. 2A is a profile view showing cell number of KCL22 cell carrying pMX-tetOFF/ PIASy-F or pMX-tetOFF introduced therein in relation to day of growth, and FIG. 2B is a profile view showing survival rate of KCL22 cell carryng pMX-tetOFF/PIASy-F or pMX-tetOFF introduced therein in relation to day of growth.
FIG. 3 is a micrograph of KCL22 cell carrying pMX-tetOFF/PIASy-F or pMX-tetOFF introduced therein.

### BEST MODE FOR CARRYING OUT THE INVENTION

Next, the present invention is described in further detail by referring to Examples of the invention which by no means limit the scope of the present invention.

### [Example 1] Preparation of AC133 positive cell

AC133-positive hematopoietic stem cells were prepared from donors by the procedure as described below. First, bone marrow aspirates were obtained from 14 donors (normal donors: 2, chronic phase patient: 7, accelerated phase patient: 2, and blast crisis phase patient: 3) after obtaining informed consent. Next, bone marrow mononuclear cells (MNC) were isolated and purified from the thus obtained bone marrow aspirates by Ficoll-Hypaque density gradient centrifugation, and the cells were washed with PBS. The bone marrow mononuclear cells were then suspended in 300 µl MACS buffer [PBS /3% fetal calf serum (FCS) /2mM EDTA], and AC133 positive cell in the bone marrow mononuclear cells were labeled with anti-AC133 MicroBeads by adding 100 µl each of FcR blocking reagent and solution of anti-AC133 antibody conjugated to magnetic beads included in the ACC133-positive cell isolation kit (Militenyi Biotec, Auburn, CA), and allowing the reaction to take place at 4°C for 30 minutes to thereby label the AC133 positive cell in the bone marrow mononuclear cell with the anti-AC133 MicroBeads. After thoroughly washing the bone marrow mononuclear cells with MACS buffer, miniMACS column (Militenyi Biotec, Auburn, CA) was used to obtain the AC133 positive cells. When the resulting AC133 positive cells were evaluated for their purity by staining with Wright-Giemsa solution (Muto Pure Chemicals), juvenile hematopoietic stem cells constituted 80% or more of the total cells in the samples from all donors.

### [Example 2] Preparation of cRNA

AC133 positive cells from each donor obtained in Example 1 were used to prepare cRNA for gene expression analysis. First, total RNA was purified from each AC133 positive cell by using RNAzol B reagent (TEL-TEST, Friendswood, TX). Next, to the resulting total RNA was added T7-dT primer:
5'-tctagtcgacggccagtgaattgtaatacgactcactatagggcgttttttttttttttttttttt-3' (SEQ ID NO: 19) to hybridize the primer to the mRNA in the total RNA, and cDNA chain complementary to the mRNA was synthesized by using reverse transcriptase Superscript II (Life Technologies, Rockville, MD). Using Superscript choice system (Life Technologies, Rockville, MD), a double-strand cDNA was then synthesized by using the resulting single strand cDNA as a template. cRNA was then synthesized from the resulting double-strand cDNA by using Ampliscribe transcription kit (Epicentre Technologies, Madison, MI). This procedure was repeated twice to finally obtain 5 to 70 µg of cRNA from the AC133 positive cells of each patient.

### [Example 3] Analysis of gene expression using DNA microarray

ExpressChip DNA microarray system (Mergen, San Leandro, CA) and the cRNA obtained in Example 2 were used to analyze the gene expression in the AC133 positive cell from each donor. Double-stranded cDNA was synthesized from 1 µg of the cRNA, and biotin-labeled cRNA was obtained from the resulting double-stranded cDNA by using Ampliscribe transcription kit (Epicentre Technologies, Madison, MI) and biotin-CTP (Life Technologies, Rockville, MD) for the substrate. Next, the labeled cRNA was hybridized with 3 types of DNA microarrays (HO-1, HO-2, and HO-3; Mergen, San Leandro, CA) each having 1152 types of oligonucleotides from human gene spotted thereon. This means that expression levels for 3456 types of genes in total were analyzed for each of the AC133 positive cell from each donor. The DNA microarray after the hybridization was brought in contact with streptavidin, and the microarray was reacted with anti-streptavidin primary antibody, and then, with Cy3 conjugated secondary antibody. It is to be noted that the streptavidin, the anti-streptavidin primary antibody, and the Cy3-conjugated secondary antibody were those purchased from Mergen (San Leandro, CA), and the experiment was carried out in accordance with the protocol attached to the ExpressChip DNA microarray system (Mergen, San Leandro, CA).

Next, fluorescence intensity of each gene spot was measured with GMS418 array scanner (Affymetrix, Santa Clara, CA), and the measurements were converted into a value by using ImaGene 3.0 (BioDiscovery, Los Angeles, CA). The resulting data were statistically processed by GeneSpring 3.2 (Silicon Genetics, Redwood, CA). To be more specific, signal intensity of all spots were digitized in every hybridization, and the values were normalized for the average spot intensity. The resulting data were subjected to cluster analysis to obtain a genetic tree (also referred to as a dendrogram) wherein genes having similar expression profile are arranged near a single tree. By this procedure, gene clusters which are active only in a particular disease phase or in a subgroup of the disease phase were found. In addition, in order to visualize the phase-specific genes in the chronic myeloid leukemia, average expression intensity of the genes was calculated for the chronic phase, the accelerated phase, and the blast crisis phase, and the genetic tree was again depicted on the bases of such values. The genes exhibiting expression level alteration in a manner specific to the chronic phase, the accelerated phase and the blast crisis phase were picked out from this average tree. The genes selected are summarized in Table 2. The unit of the expressed amount shown in Table 2 is arbitrary, and the values of 0.6 or less means that the expression was below the detection limit. The values are not absolute, and may vary by the conditions of the patient, preparation of the mRNA, and the conditions of the DNA microarray.

**Table 2**

| Genes which showed alteration in the expression level | | | | |
|---|---|---|---|---|
| Amount expressed (Arbitrary unit) | Normal | Chronic phase | Accelerated phase | Blast crisis phase |
| IFIT-2 | 17.8 | 128.7 | 7.9 | 3.8 |
| LAGE-1 | < 0.6 | 20.1 | < 0.6 | <0.6 |
| BAGE | < 0.6 | 19.0 | < 0.6 | <0.6 |
| DDB1 | 1.0 | 4.5 | 17.3 | 23.9 |
| ETS-2 | 1.5 | 2.0 | 4.7 | 7.5 |
| PIASy | 26.1 | 52.4 | 4.2 | 10.0 |
| PIASx-α,β | 5.8 | 16.8 | 1.7 | 1.5 |
| DAPK3 | 63.7 | 109.4 | 9.2 | 20.7 |

39 genes were found to exhibit specific expression in the chronic phase. Among such chronic phase-specific genes, the most notable was interferon-induced protein with tetratricopeptide repeats 2 (IFIT-2) gene (SEQ ID NO: 1). Transcription of the IFIT-2 gene was very active in the 5 samples (CP-3 to CP-7) out of the 7 samples taken from chronic phase donors (CP-1 to CP-7), while very weak or no transcription was found in the samples taken from normal donors or chronic myeloid leukemia patients of other disease phase. In samples CP-6 and CP-7, LAGE-1 gene (SEQ ID NO: 3) and B melanoma antigen (BAGE) gene (SEQ ID NO: 5) were also found to exhibit specific expression.

By using the same average tree, attempt was made to identify genes whose expression is not found in the AC 133 positive cells from a normal donor or a patient of chronic phase while the expression is induced in the AC133 positive cell from the patient of accelerated phase or blast crisis phase. As a result, DNA damage binding protein (DDB1) gene (SEQ ID NO: 7) and ETS2 transcription factor gene (SEQ ID NO: 9) were found. DDB1 is involved in the DNA repair mechanism, and loss of its activity is related to complementation group E of xeroderma pigmentosum. Loss of stability of the DNA and various types of chromosome aberration are often found in the blast from chronic myeloid leukemia patient of advanced phase. Accordingly, the induction of the DDB1 gene expression as seen here is believed to reflect the DNA damage accumulated in the leukemia cell of the patients in the accelated phase or the blast crisis phase. In addition, both DDB1 gene and ETS2 gene are induced in CP-1 patient, indicating the possibility that this patient is in the course of the transfer to the accelerated phase or the blast crisis phase.

By using the same average tree, attempt was also made to identify genes whose expression is reduced with the phase transfer to the accelerated phase or the blast crisis phase. 21 genes were then found to exhibit high expression in the AC133 positive cells taken from normal donors or patients in their chronic phase while the expression is reduced in the AC133 positive cells taken from patients in their accelerated phase or blast crisis phase. Among such genes, PIASy gene (SEQ ID NO: 11), PIASx-α gene (SEQ ID NO: 13), and PIASx-β gene (SEQ ID NO: 15) exhibited most remarkable reduction. PIAS1 and PIAS3 bind to STAT1 and STAT3, respectively, and suppress their activity. Accodingly, although little is known for a protein to which PIASy binds, it is postulated that PIASy may have some function to regulate a member of STAT pathway proteins. It is known that STAT protein is frequently phosphorylated in excess and is activated in the cells derived from individuals suffering from various types of leukemia. Activation of the STAT protein occurs upon introduction of the BCR-ABL kinase into the hematopoietic system, and this indicates the possibility of the STAT protein being a mediator of the BCR-ABL signaling. It was thus speculated that overexpression of PIASy hinders transport of the activated STAT proteins in CML blast cells in chronic phase, while loss of PIASy results in uncontrolled blast cell growth that leads to the progress of the disease phase.

### [Example 4] Confirmation of expression level by real time PCR

For the IFIT-2 gene and the PIASy gene which had been identified to be the phase-specific genes in chronic myeloid leukemia in Example 3, real time PCR was carried out to confirm their expression level in the AC133 positive cells from normal donors and patients of each disease phases. First, cDNA was prepared from the AC133 positive cells of patients by the procedure as described in Examples 1 and 2. The resulting cDNA was subjected to PCR using SYBR Green PCR Core Reagents (PE Applied Biosystems, Foster City, CA). In the PCR, the primers used for the amplification IFIT-2 gene were 5'-ctgcggtatggcaactttcag-3' (SEQ ID NO: 20) and 5'-aggaatgccaagacatgcaa-3' (SEQ ID NO: 21), and the primers used for the amplification of PIASy gene were 5'-aactacggcaagagctactcggtg-3' (SEQ ID NO: 22) and 5'-gttcatctgcaggtagaagacggc-3' (SEQ ID NO: 23). β-actin gene was also used for the control according to the method used in the art. ABI PRISM 7700 Sequence Detection System (PE Applied Biosystems) was used to determine the cycle threshold (C_{T} value) at which the exponential growth of the PCR product is first detected in the real time monitoring of the incorporation of the SYBR Green dye to the PCR product. Amount of the target gene in relation to the β-actin mRNA amount was calculated from the C_{T} value of the cDNA corresponding to the β-actin gene and the target gene. The amount of IFIT-2 gene and PIASy gene expressed in the AC133 positive cells from the patients of each disease phase was then found to be consistent with the measurement of Example 3 obtained by using a DNA microarray confirming the reproducibility in the expression of these genes.

### [Example 5] Effects of PIASy gene introduction

PIASy gene which had been identified in Example 3 to be a phase-specific gene for the chronic myeloid leukemia was introduced in a cell from a chronic myeloid leukemia patient of blast crisis phase to thereby examine the effects of the PIASy gene expression.

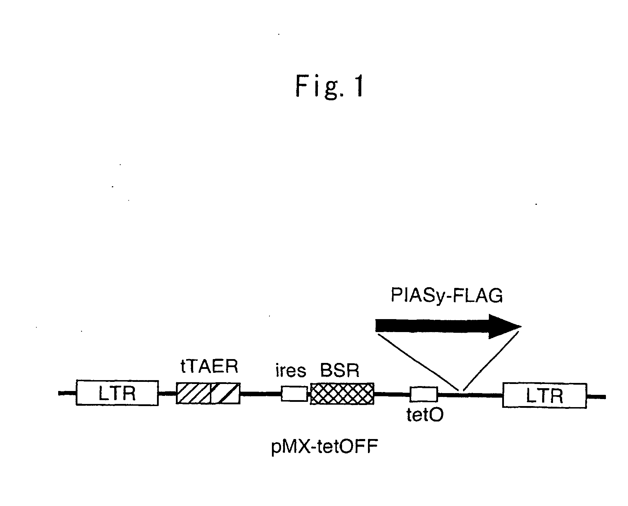

The expression vector containing the PIASy gene was constructed as described below. First, pMX-tetOFF vector was prepared by introducing the cDNA coding for tTAER and ires-BSR and tetO fragments in pMX retroviral vector (Onishi et al.: Exp. Hematol., 24,: 324-329 (1996)). Next, PIASy-F gene coding for PIASy (PIASy-F) having FLAG epitope tag (Eastman Kodak) conjugated at the C terminal was amplified by the PCR using PIASy cDNA (Helix Research Institute) for the template. PIASy-F gene was then ligated to the downstream of the tetO fragment in the pMX-tetOFF vector to thereby construct the expression vector (pMX-tetOFF/PIASy-F) (FIG. 1). The resulting expression vector was introduced in a packaging cell line (PA317, American Type Culture Collection).

In this Example, KCL22 cells (American Type Culture Collection) were used for the cell from a chronic myeloid leukemia patient in the blast crisis phase. KCL22 cells were infected with the pMX-tetOFF/PIASy-F in the culture supernatant of the PA317 by treating the cell in RPMI1640 medium supplemented with 10% fetal bovine serum (FBS) for 24 hours in the presence RetroNectin (Takara Shuzo). The KCL22 cells recovered were then cultivated in the RPMI1640 medium supplemented with 10% fetal bovine serum (FBS) further supplemented with 5 µg/ml of blasticidin and 1 µg/ml of tetracycline. PIASy-F gene in the expression vector was then expressed by cultivating the KCL22 cells in the RPMI1640 medium supplemented with 10% fetal bovine serum (FBS) having 2 µM of 17β-estradiol added thereto.

It is to be noted that the expression vector (pMX-tetOFF/PIASy-F) shown in FIG. 1 does not express the gene (PIASy-F gene) in the downstream of tetO when tetracycline is present and β estradiol is absent. In contrast, the expression vector (pMX-tetOFF/PIASy-F) expresses the gene (PIASy-F gene) in the downstream of tetO when tetracycline is absent and β estradiol is present. The effects of the PIASy gene expression was confirmed by examining the growth of KCL22 cells carrying pMX-tetOFF/PIASy-F introduced therein.

To be more specific, the KCL22 cells carrying pMX-tetOFF introduced therein and the KCL22 cells carrying pMX-tetOFF/PIASy-F introduced therein were evaluated for their cell number and the survival rate both under the conditions that allow expression of the gene in the downstream of tetO and under the conditions that do not allow expression of the gene in the downstream of tetO. It is to be noted that KCL22 cells cultivated in RPMI1640 medium supplemented with 10% fetal bovine serum (FBS) in both conditions.

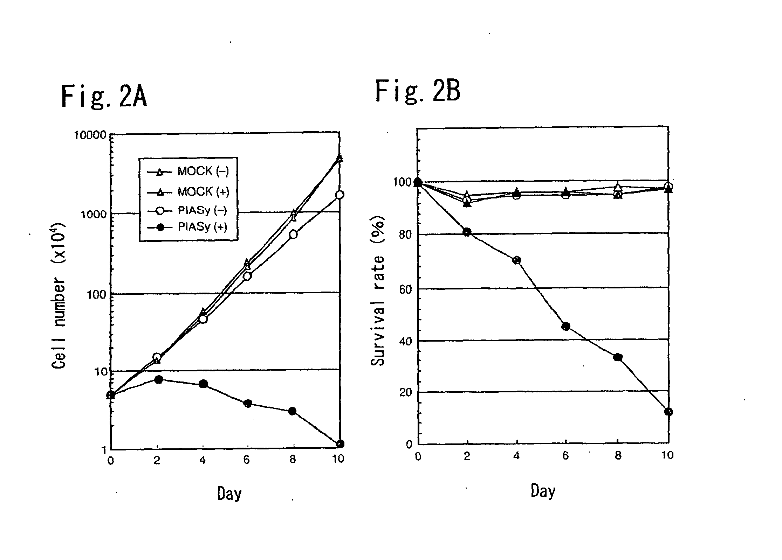

The results of evaluating the cell number and the survival rate are shown in FIGS. 2A and 2B, respectively. As shown in FIGS.2A and 2B, both the cell number and the survival rate decreased in proportion to the growing period of the KCL22 cells expressing PIASy gene. On the contrary, the cell number and the survival rate for the KCL22 cells carrying pMX-tetOFF/PIASy-F, which show no expression of the PIASy gene ("PIASy (-)" in FIG.2), were not different from those for the KCL22 cells carrying pMX-tetOFF irrespective of the growing period of the cells.

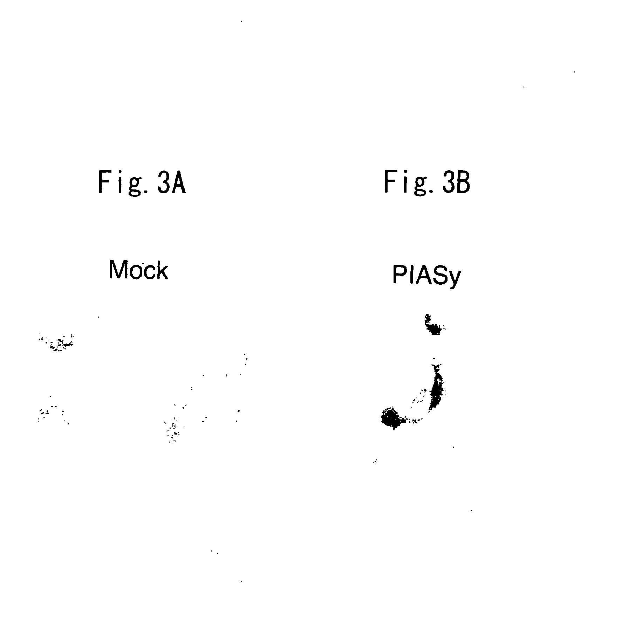

FIGS. 3A and 3B are photomicrographs (x 400) of the KCL22 cells carrying pMX-tetOFF introduced therein and the KCL22 cells carrying pMX-tetOFF/PIASy-F introduced therein when these cells were cultivated for 6 days under the conditions that allow expression of the gene in the downstream of tetO. FIG. 3A is the photomicrograph of the KCL22 cells carrying pMX-tetOFF introduced therein (MOCK), and FIG. 3B is the photomicrograph of the KCL22 cells carrying pMX-tetOFF/PIASy-F introduced therein (PIASy). It is to be noted that both KCL22 cells were stained with Wright-Giemsa solution. As shown in FIGS. 3A and 3B, the KCL22 cells carrying pMX-tetOFF introduced therein showed normal phenotype (intermediate sized cell having a basophilic cytoplasm, high proportion of nucleus in relation to cytoplasm, and good nuclear structure) while the KCL22 cells carrying pMX-tetOFF/PIASy-F introduced therein (PIASy) showed apoptotic change (size reduction of the cell, and contraction and fragmentation of the nucleus).

FIGS. 2 and 3 revealed that expression of the PIASy-F gene in the KCL22 cells carrying pMX-tetOFF/PIASy-F introduced therein results in the induction of apoptosis in the KCL22 cells. PIASy protein has been recently demonstrated to inhibit activity of STAT1, and it is postulated that the apoptosis as described above is induced by the inhibition of the STAT1 in the KCL22 cells. High-level expression of the PIASy gene has been observed in the AC133 positive cells derived from each of normal donors and chronic patients, and it may relate to the reduced risk of chronic patients to progress into the blast crisis phase.

### INDUSTRIAL APPLICABILITY

Provided by this invention are a method for detecting chronic myeloid leukemia; a method for evaluating a compound for its effectiveness in treating or preventing chronic myeloid leukemia; a compound which is effective in treating or preventing chronic myeloid leukemia containing the compound obtained by such evaluation method; a therapeutic or prophylactic agent for chronic myeloid leukemia containing such compound; and a method for determining the risk of being susceptible for chronic myeloid leukemia.

## Claims

1. A method for detecting chronic myeloid leukemia comprising the step of measuring expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3 gene in a test tissue or a test cell; or determining expression profile of a gene group containing at least one of said genes so as to thereby detect chronic myeloid leukemia.

2. A method for detecting chronic myeloid leukemia according to claim 1 wherein the test cell is a bone marrow cell or a blood cell.

3. A method for detecting chronic myeloid leukemia according to claim 2 wherein the bone marrow cell or the blood cell is pluripotent hematopoietic stem cell.

4. A method for detecting chronic myeloid leukemia according to claim 3 wherein the pluripotent hematopoietic stem cell is a AC133 protein-positive cell.

5. A method for detecting chronic myeloid leukemia according to claim 1 wherein the measurement of the gene expression level or the determination of the expression profile of the gene group is carried out at the level of mRNA.

6. A method for detecting chronic myeloid leukemia according to claim 5 wherein the measurement of the gene expression level or the determination of the expression profile of the gene group at the level of mRNA is carried out by means of gene amplification and/or by using a DNA microarray.

7. A method for detecting chronic myeloid leukemia according to claim 1 wherein the measurement of the gene expression level or the determination of the expression profile of the gene group is carried out at the level of protein.

8. A method for detecting chronic myeloid leukemia according to claim 7 wherein the measurement of the gene expression level or the determination of the expression profile of the gene group at the level of protein is carried out by means of immunological method and/or by using a protein chip.

9. A method for determining phase of chronic myeloid leukemia wherein the phase of the chronic myeloid leukemia is determined by using the detection method of chronic myeloid leukemia according to any one of claims 1 to 8.

10. A method for determining phase of chronic myeloid leukemia according to claim 9 wherein the phase of the chronic myeloid leukemia is selected from the group consisting of chronic phase, accelerated phase, and blastic phase.

11. A method for evaluating a compound for its effectiveness in treating or preventing chronic myeloid leukemia, comprising the steps of:
(a) administering the compound to be evaluated to a test animal or a test cell, or bringing the compound in contact with a test animal or a test cell; and
(b) confirming whether the compound regulates expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3 gene, or a gene which is functionally equivalent to such gene in test animal or the test cell.

12. A method for evaluating a compound for its effectiveness in treating or preventing chronic myeloid leukemia, comprising the steps of:
(a) bringing the compound to be evaluated in contact with a test animal or a test cell containing a fusion gene of an expression-regulatory region for a gene selected from the group consisting of IFIT-2 gene, LAGE-1 gene, BAGE gene, DDB1 gene, ETS2 gene, PIASy gene, PIASx-α gene, PIASx-β gene, and DAPK3 gene; and a reporter gene; and
(b) measuring the level of the reporter gene expressed in the test animal or the test cell to evaluate the compound.

13. A method for evaluating a compound for its effectiveness in treating or preventing chronic myeloid leukemia, comprising the steps of:
(a) bringing the compound to be evaluated in contact with a protein encoded by a gene selected from the group consisting of IFIT-2 gene, LAGE-1 gene, BAGE gene, DDB1 gene, ETS2 gene, PIASy gene, PIASx-α gene, PIASx-β gene, and DAPK3 gene; and
(b) measuring the activity of the protein to evaluate the compound.

14. A method for evaluating a compound for its effectiveness in treating or preventing chronic myeloid leukemia, comprising the steps of:
(a) bringing the compound to be evaluated in contact with a STAT protein whose activity is inhibited by a protein encoded by PIASy gene, PIASx-α gene, or PIASx-β gene; and
(b) measuring the activity of the STAT protein to evaluate the compound.

15. A therapeutic or prophylactic agent for chronic myeloid leukemia comprising a compound obtained by the evaluation method of any one of claims 11 to 14 as an active ingredient.

16. A reagent for research purpose, or an agent for detection, treatment, or prevention of chronic myeloid leukemia comprising an antibody directed against a protein encoded by a gene selected from the group consisting of IFIT-2 gene, LAGE-1 gene, BAGE gene, DDB1 gene, ETS2 gene, PIASy gene, PIASx-α gene, PIASx-β gene, and DAPK3 gene.

17. A method for evaluating the risk of being susceptible for chronic myeloid leukemia comprising the step of detecting a genetic polymorphism which causes abnormality in expression level of a gene selected from the group consisting of IFIT-2 gene, LAGE-1 gene, BAGE gene, DDB1 gene, ETS2 gene, PIASy gene, PIASx-α gene, PIASx-β gene, and DAPK3.

18. A method for evaluating the risk of being susceptible to chronic myeloid leukemia comprising the step of detecting a genetic polymorphism which causes abnormality in biological activity of the protein encoded by a gene selected from the group consisting of IFIT-2 gene, LAGE-1 gene, BAGE gene, DDB1 gene, ETS2 gene, PIASy gene, PIASx-α gene, PIASx-β gene, and DAPK3 gene.

19. A therapeutic or prophylactic agent for chronic myeloid leukemia comprising an antisense nucleic acid which is capable of specifically hybridizing with mRNA of a gene selected from the group consisting of IFIT-2 gene, LAGE-1 gene, BAGE gene, DDB1 gene, and ETS2 gene.

20. A therapeutic or prophylactic agent for chronic myeloid leukemia comprising a ribozyme capable of specifically cleaving mRNA of a gene selected from the group consisting of IFIT-2 gene, LAGE-1 gene, BAGE gene, DDB1 gene, and ETS2 gene.
